# EUROPEAN PATENT APPLICATION

(11) **EP 1 484 401 A1**
(43) Date of publication of application: **08.12.2004**
(21) Application number: 04020519.7
(22) Date of filing: 20.07.2000
(51) Int. Cl.: C12N 15/12, C07K 14/47, C07K 14/575, C07K 14/705, A61K 38/17, C07K 16/18, C07K 16/28, G01N 33/53, G01N 33/68

(54) **Secreted polypeptides and corresponding polynucleotides**

(30) Priority: 20.07.1999 US 144722 P; 29.11.1999 US 167785 P; 19.07.2000 US 619252
(62) Divisional of application: 00948854.5
(71) Applicant: Curagen Corporation, New Haven, CT 06511 (US)
(72) Inventor: Shimkets, Richard A., West Haven, CT 06516 (US); Fernandes, Elma, Branford, CT 06405 (US)
(74) Representative: MacLean, Martin Robert

(57) **Abstract**

The invention provides polypeptides, designated herein as SECP polypeptides, as well as polynucleotides encoding SECP polypeptides, and antibodies that immunospecifically-bind to SECP polypeptide or polynucleotide, or derivatives, variants, mutants, or fragments thereof. The invention additionally provides methods in which the SECP polypeptide, polynucleotide, and antibody are used in the detection, prevention, and treatment of a broad range of pathological states.

## Description

### FIELD OF THE INVENTION

The invention relates to generally to polynucleotides and the polypeptides encoded thereby and more particularly to polynucleotides encoding polypeptides that cross one or more membranes in eukaryotic cells.

### BACKGROUND OF THE INVENTION

Eukaryotic cells are subdivided by membranes into multiple, functionally-distinct compartments, referred to as organelles. Many biologically important proteins are secreted from the cell after crossing multiple membrane-bound organelles. These proteins can often be identified by the presence of sequence motifs referred to as "sorting signals" in the protein, or in a precursor form of the protein. These sorting signals can also aid in targeting the proteins to their appropriate destination.

One specific type of sorting signal is a signal sequence, which is also referred to as a signal peptide or leader sequence. This signal sequence, which can be present as an amino-terminal extension on a newly synthesized polypeptide. A signal sequence possesses the ability to "target" proteins to an organelle known as the endoplasmic reticulum (ER).

The signal sequence takes part in an array of protein-protein and protein-lipid interactions that result in the translocation of a signal sequence-containing polypeptide through a channel within the ER. Following translocation, a membrane-bound enzyme, designated signal peptidase, liberates the mature protein from the signal sequence.

Secreted and membrane-bound proteins are involved in many biologically diverse activities. Examples of known, secreted proteins include, e.g., insulin, interferon, interleukin, transforming growth factor-β, human growth hormone, erythropoietin, and lymphokine. Only a limited number of genes encoding human membrane-bound and secreted proteins have been identified.

### SUMMARY OF THE INVENTION

The invention is based, in part, upon the discovery of novel nucleic acids and secreted polypeptides encoded thereby. The nucleic acids and polypeptides are collectively referred to herein as "SECP".

Accordingly, in one aspect, the invention includes an isolated nucleic acid that encodes a SECP polypeptide, or a fragment, homolog, analog or derivative thereof. For example, the nucleic acid can encode a polypeptide at least 85% identical to a polypeptide comprising the amino acid sequences of SEQ ID NO:2, 4, 6, 8, 10, 12, 14, 16, and 18. The nucleic acid can be, *e*.*g*., a genomic DNA fragment, cDNA molecule. In some embodiments, the nucleic acid includes the sequence the invention provides an isolated nucleic acid molecule that includes the nucleic acid sequence of any of SEQ ID NO:1, 3, 5, 7, 9, 11, 13, 15, and 17.

Also included within the scope of the invention is a vector containing one or more of the nucleic acids described herein, and a cell containing the vectors or nucleic acids described herein.

The invention is also directed to host cells transformed with a vector comprising any of the nucleic acid molecules described above.

In another aspect, the invention includes a pharmaceutical composition that includes a SECP nucleic acid and a pharmaceutically acceptable carrier or diluent.

In a further aspect, the invention includes a substantially purified SECP polypeptide, *e*.*g*., any of the SECP polypeptides encoded by a SECP nucleic acid, and fragments, homologs, analogs, and derivatives thereof. The invention also includes a pharmaceutical composition that includes a SECP polypeptide and a pharmaceutically acceptable carrier or diluent.

In a still a further aspect, the invention provides an antibody that binds specifically to a SECP polypeptide. The antibody can be, *e*.*g*., a monoclonal or polyclonal antibody, and fragments, homologs, analogs, and derivatives thereof. The invention also includes a pharmaceutical composition including SECP antibody and a pharmaceutically acceptable carrier or diluent. The invention is also directed to isolated antibodies that bind to an epitope on a polypeptide encoded by any of the nucleic acid molecules described above.

The invention also includes kits comprising any of the pharmaceutical compositions described above.

The invention further provides a method for producing a SECP polypeptide by providing a cell containing a SECP nucleic acid, e.g., a vector that includes a SECP nucleic acid, and culturing the cell under conditions sufficient to express the SECP polypeptide encoded by the nucleic acid. The expressed SECP polypeptide is then recovered from the cell. Preferably, the cell produces little or no endogenous SECP polypeptide. The cell can be, *e*.*g*., a prokaryotic cell or eukaryotic cell.

The invention is also directed to methods of identifying a SECP polypeptide or nucleic acids in a sample by contacting the sample with a compound that specifically binds to the polypeptide or nucleic acid, and detecting complex formation, if present.

The invention further provides methods of identifying a compound that modulates the activity of a SECP polypeptide by contacting SECP polypeptide with a compound and determining whether the SECP polypeptide activity is modified.

The invention is also directed to compounds that modulate SECP polypeptide activity identified by contacting a SECP polypeptide with the compound and determining whether the compound modifies activity of the SECP polypeptide, binds to the SECP polypeptide, or binds to a nucleic acid molecule encoding a SECP polypeptide.

In a another aspect, the invention provides a method of determining the presence of or predisposition of a SECP-associated disorder in a subject. The method includes providing a sample from the subject and measuring the amount of SECP polypeptide in the subject sample. The amount of SECP polypeptide in the subject sample is then compared to the amount of SECP polypeptide in a control sample. An alteration in the amount of SECP polypeptide in the subject protein sample relative to the amount of SECP polypeptide in the control protein sample indicates the subject has a tissue proliferation-associated condition. A control sample is preferably taken from a matched individual, *i*.*e*., an individual of similar age, sex, or other general condition but who is not suspected of having a tissue proliferation-associated condition. Alternatively, the control sample may be taken from the subject at a time when the subject is not suspected of having a tissue proliferation-associated disorder. In some embodiments, the SECP is detected using a SECP antibody.

In a further aspect, the invention provides a method of determining the presence of or predisposition of a SECP-associated disorder in a subject. The method includes providing a nucleic acid sample (*e*.*g*., RNA or DNA, or both) from the subject and measuring the amount of the SECP nucleic acid in the subject nucleic acid sample. The amount of SECP nucleic acid sample in the subject nucleic acid is then compared to the amount of a SECP nucleic acid in a control sample. An alteration in the amount of SECP nucleic acid in the sample relative to the amount of SECP in the control sample indicates the subject has a tissue proliferation-associated disorder.

In a still further aspect, the invention provides method of treating or preventing or delaying a SECP-associated disorder. The method includes administering to a subject in which such treatment or prevention or delay is desired a SECP nucleic acid, a SECP polypeptide, or a SECP antibody in an amount sufficient to treat, prevent, or delay a tissue proliferation-associated disorder in the subject.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. In the case of conflict, the present Specification, including definitions, will control. In addition, the materials, methods, and examples are illustrative only and not intended to be limiting.

Other features and advantages of the invention will be apparent from the following detailed description and claims.

### BRIEF DESCRIPTION OF THE FIGURES

FIG. 1 is a representation of a SECP 1 nucleic acid sequence (SEQ ID NO:1) according to the invention, along with an amino acid sequence (SEQ ID NO:2) encoded by the nucleic acid sequence.
FIG. 2 is a representation of a SECP 2 nucleic acid sequence (SEQ ID NO:3) according to the invention, along with an amino acid sequence (SEQ ID NO:4) encoded by the nucleic acid sequence.
FIG. 3 is a representation of a SECP 3 nucleic acid sequence (SEQ ID NO:5) according to the invention, along with an amino acid sequence (SEQ ID NO:6) encoded by the nucleic acid sequence.
FIG. 4 is a representation of a SECP 4 nucleic acid sequence (SEQ ID NO:7) according to the invention, along with an amino acid sequence (SEQ ID NO:8) encoded by the nucleic acid sequence.
FIG. 5 is a representation of a SECP 5 nucleic acid sequence (SEQ ID NO:9) according to the invention, along with an amino acid sequence (SEQ ID NO:10) encoded by the nucleic acid sequence.
FIG. 6 is a representation of a SECP 6 nucleic acid sequence (SEQ ID NO:11) according to the invention, along with an amino acid sequence (SEQ ID NO:12) encoded by the nucleic acid sequence.
FIG. 7 is a representation of a SECP 7 nucleic acid sequence (SEQ ID NO:13) according to the invention, along with an amino acid sequence (SEQ ID NO:14) encoded by the nucleic acid sequence.
FIG. 8 is a representation of a SECP 8 nucleic acid sequence (SEQ ID NO:15) according to the invention, along with an amino acid sequence (SEQ ID NO:16) encoded by the nucleic acid sequence.
FIG. 9 is a representation of a SECP 9 nucleic acid sequence (SEQ ID NO:17) according to the invention, along with an amino acid sequence (SEQ ID NO:18) encoded by the nucleic acid sequence.
FIG. 10 is a representation of an alignment of the proteins encoded by clones 11618130.0.27 (SEQ ID NO:4) and 11618130.0.184 (SEQ ID NO:16).
FIG. 11 is a representation of an alignment of the proteins encoded by clones 14578444.0.143 (SECP4; SEQ ID NO:8) and 14578444.0.47 (SECP 5; SEQ ID NO:10).
FIG. 12 is a representation of a Western blot of a polypeptide expressed in 293 cells of a polynucleotide containing sequences encoded by clone 11618130.
FIG. 13 is a representation of a Western blot of a polypeptide expressed in 293 cells of a polynucleotide containing sequence encoded by clone 16406477.
FIG. 14 is a representation of a real-time expression analysis of the clones of the invention.

### DETAILED DESCRIPTION OF THE INVENTION

The invention provides novel polynucleotides and the polypeptides encoded thereby. Included in the invention are ten novel nucleic acid sequences and their encoded polypeptides. These sequences are collectively referred to as "SECP nucleic acids" or "SECP polynucleotides" and the corresponding encoded polypeptide is referred to as a "SECP polypeptide" or "SECP protein". For example, a SECP nucleic acid according to the invention is a nucleic acid including a SECP nucleic acid, and a SECP polypeptide according to the invention is a polypeptide that includes the amino acid sequence of a SECP polypeptide. Unless indicated otherwise, "SECP" is meant to refer to any of the novel sequences disclosed herein. Each of the nucleic acid and amino acid sequences have been assigned a unique SECP Identification Number, with designations SECP1 through SECP9.

TABLE 1 provides a cross-reference to the assigned SECP Number, Clone or Probe Identification Number, and Sequence Identification Number (SEQ ID NO:) for both the nucleic acid and encoded polypeptides of SECP1-9.

**TABLE 1**

| **CLONE/PROBE** | **FIGURE** | SEQ ID NO: (Nucleic Acid) | SEQ ID NO: (Polypeptide) |
|---|---|---|---|
| 21433858 | 1 | 1 | 2 |
| 11618130.0.27 | 2 | 3 | 4 |
| 11696905-0-47 | 3 | 5 | 6 |
| 14578444.0.143 | 4 | 7 | 8 |
| 14578444.0.47 | 5 | 9 | 10 |
| 14998905.0.65 | 6 | 11 | 12 |
| 16406477.0.206 | 7 | 13 | 14 |
| 11618130.0.184 | 8 | 15 | 16 |
| 21637262.0.64 | 9 | 17 | 18 |
| 11618130 Forward | | 19 | |
| 11618130 Reverse | | 20 | |
| PSec-V5-His Forward | | 21 | |
| PSec-V5-His Reverse | | 22 | |
| 16406477 Forward | | 23 | |
| 16406477 Reverse | | 24 | |
| Ag 383 (F) | | 25 | |
| Ag 383 (R) | | 26 | |
| Ag 383 (P) | | 27 | |
| Ag 53 (F) | | 28 | |
| Ag 53 (R) | | 29 | |
| Ag 53 (P) | | 30 | |
| Ag 127 (F) | | 31 | |
| Ag 127 (R) | | 32 | |
| Ag 127 (P) | | 33 | |
| Ab 5(F) | | 34 | |
| Ab 5(R) | | 35 | |
| Ab 5(P) | | 36 | |

Nucleic acid sequences and polypeptide sequences for SECP nucleic acids and polypeptides, as disclosed herein, are provided in the following section of the Specification.

SECP nucleic acids, and their encoded polypeptides, according to the invention are useful in a variety of applications and contexts. For example, various SECP nucleic acids and polypeptides according to the invention are useful, *inter alia,* as novel members of the protein families according to the presence of domains and sequence relatedness to previously described proteins.

SECP nucleic acids and polypeptides according to the invention can also be used to identify cell types based on the presence or absence of various SECP nucleic acids according to the invention. Additional utilities for SECP nucleic acids and polypeptides are discussed below.

### SECP1

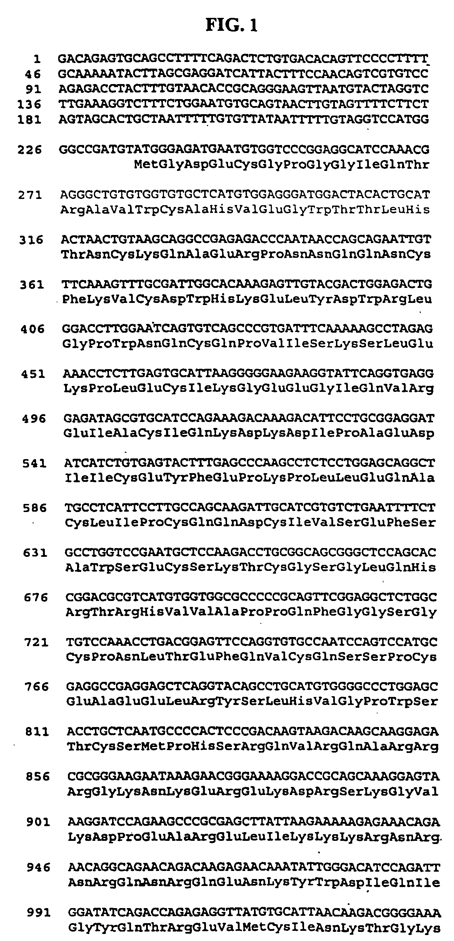

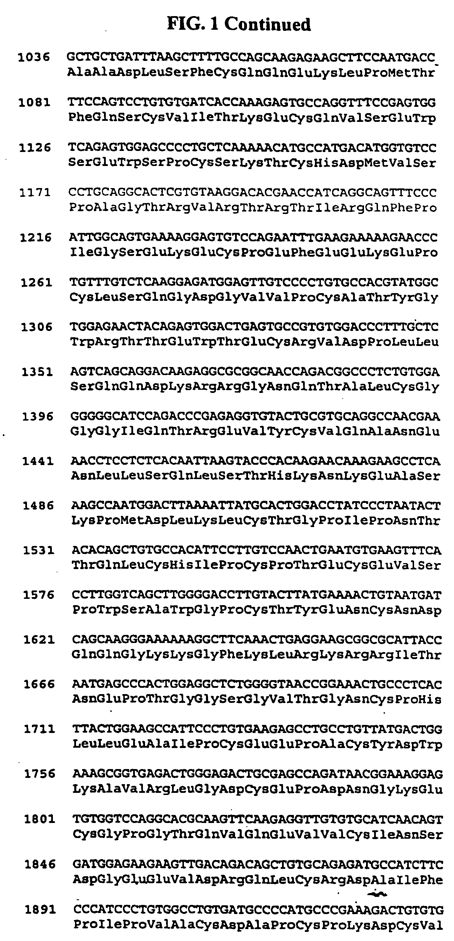

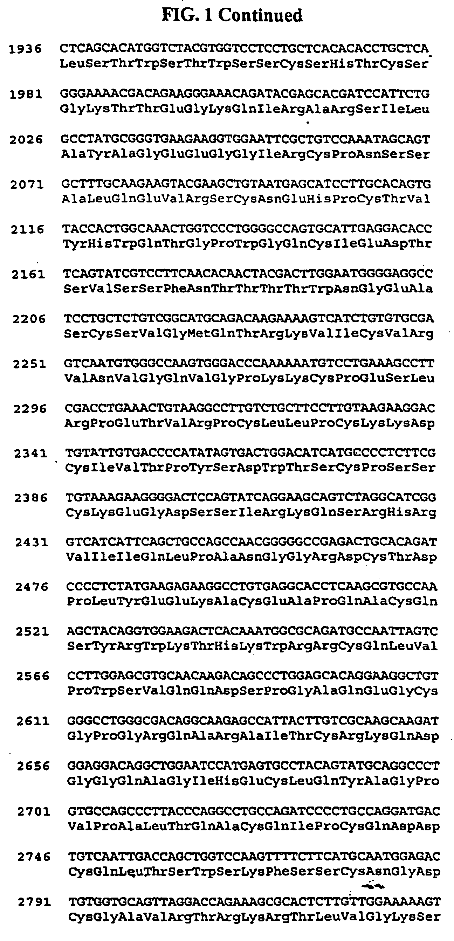

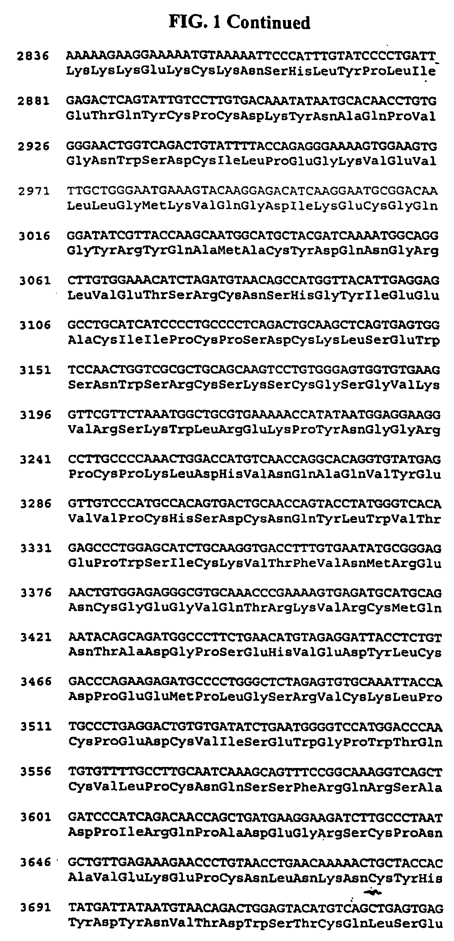

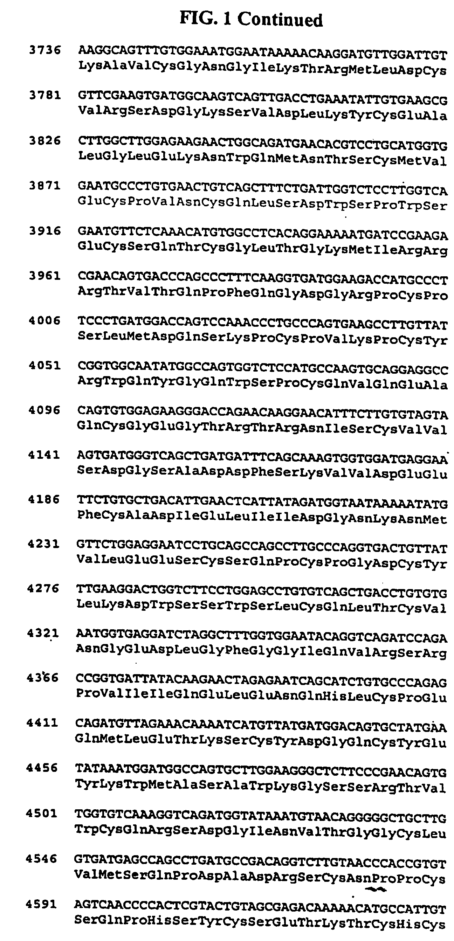

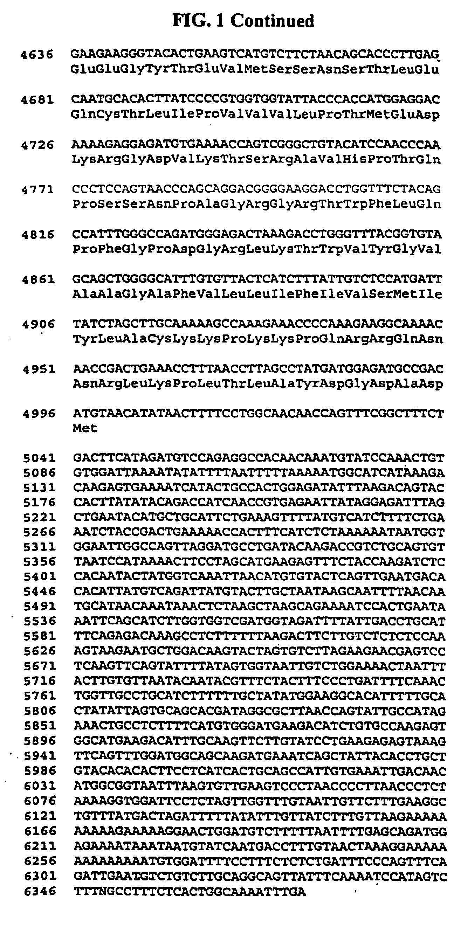

A SECP1 nucleic acid and polypeptide according to the invention includes the nucleic acid sequence (SEQ ID NO:1) and encoded polypeptide sequence (SEQ ID NO:2) of clone 21433858. FIG. 1 illustrates the nucleic acid and amino acid sequences, as well as the alignment between these two sequences.

This clone includes a nucleotide sequence (SEQ ID NO:1) of 6373 bp. The nucleotide sequence includes an open reading frame (ORF) encoding a polypeptide of 1588 amino acid residues (SEQ ID NO:2) with a predicted molecular weight of 178042.1 Daltons. The start codon is located at nucleotides 235-237 and the stop codon is located at nucleotides 4999-5001. The protein encoded by clone 21433858 is predicted by the PSORT program to localize in the plasma membrane with a certainty of 0.7300. The program SignalP predicts that there is a signal peptide with the most probable cleavage site located between residues 23 and 24, in the sequence CMG-DE.

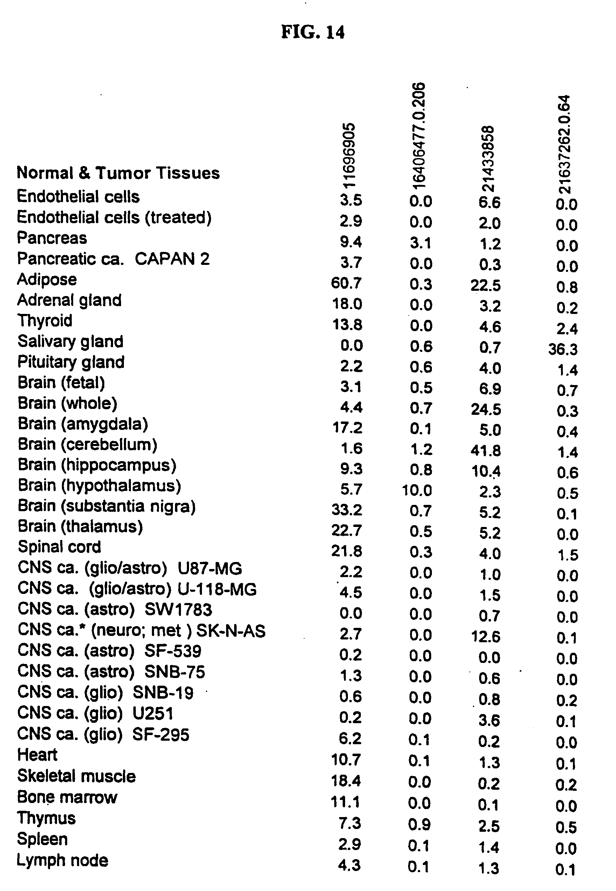

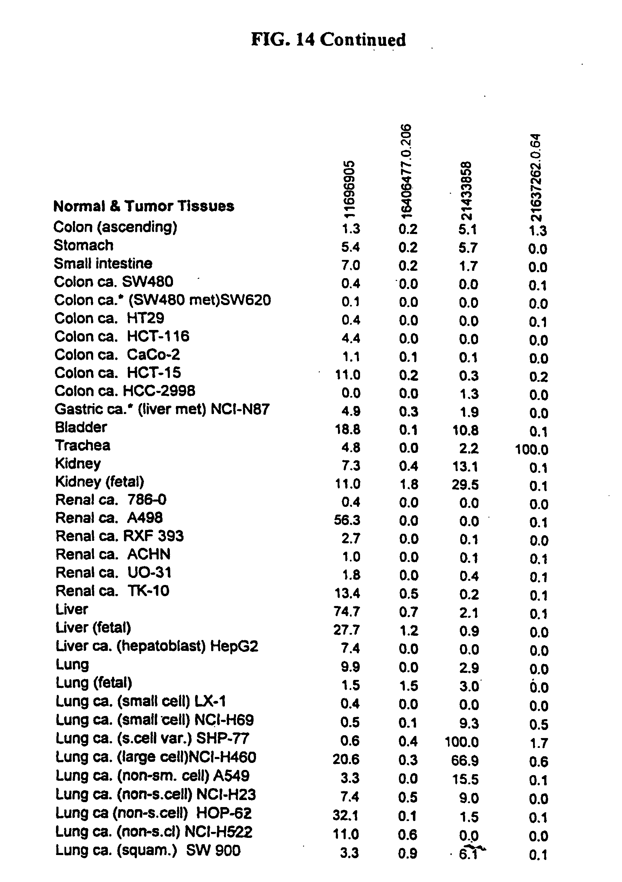

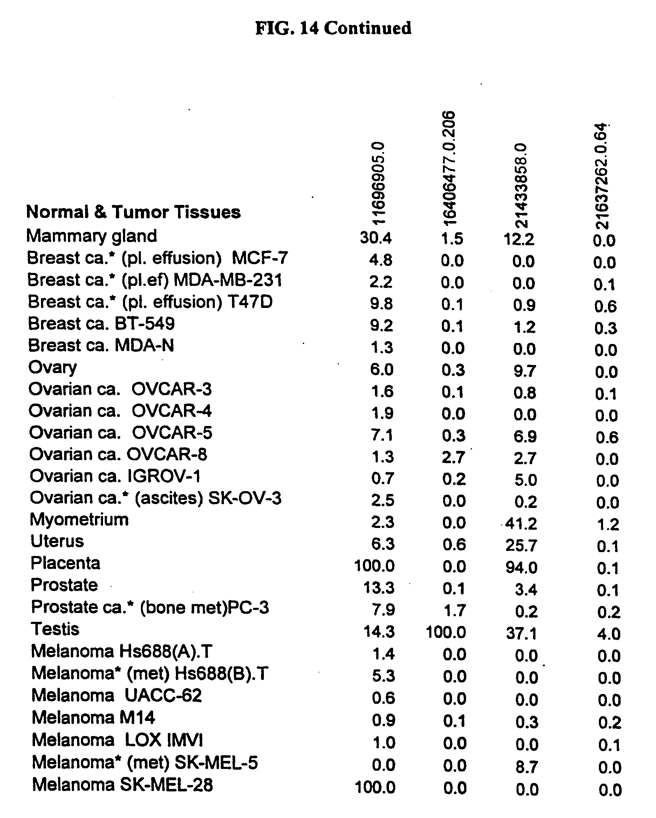

Real-time gene expression analysis was performed on SECP1 (clone 21433858). The results demonstrate that RNA sequences with homology to clone 21433858 are detected in various cell types. The relative abundance of RNA homologous to clone 21433858 is shown in FIG. 14 (see also Examples, below). Cell types endothelial cells (treated and untreated), pancreas, adipose, adrenal gland, thyroid, mammary gland, myometrium, uterus, placenta, prostate, testis, and in neoplastic cells derived from ovarian carcinoma OVCAR-3, ovarian carcinoma OVCAR-5, ovarian carcinoma OVCAR-8, ovarian carcinoma IGROV-1, ovarian carcinoma (ascites) SK-OV-3, breast carcinoma BT-549, prostate carcinoma (bone metastases) PC-3, Melanoma M14, and melanoma (met) SK-MEL-5. Accordingly, SECP1 nucleic acids according to the invention can be used to identify one or more of these cell types. The presence of RNA sequences homologous to a SECP1 nucleic in a sample indicates that the sample contains one or more of the above-cell types.

A search of sequence databases using BLASTX reveals that residues 299-1588 of the polypeptide encoded clone 21433858 are 100% identical to the 1290 residue human KIAA0960 protein (ACC: SPTREMBL-ACC:Q9UPZ6). In addition, the protein of clone 21433858 has 542 of 543 residues (99%) identical to, and 543 of 543 residues (100%) positive with, the 543 residue fragment of a human hypothetical protein (SPTREMBL-ACC:060407).

The proteins of the invention encoded by clone 21433858 include the protein disclosed as being encoded by the ORF described herein, as well as any mature protein arising therefrom as a result of post-translational modifications. Thus, the proteins of the invention encompass both a precursor and any active forms of the clone 21433858 protein.

### SECP2

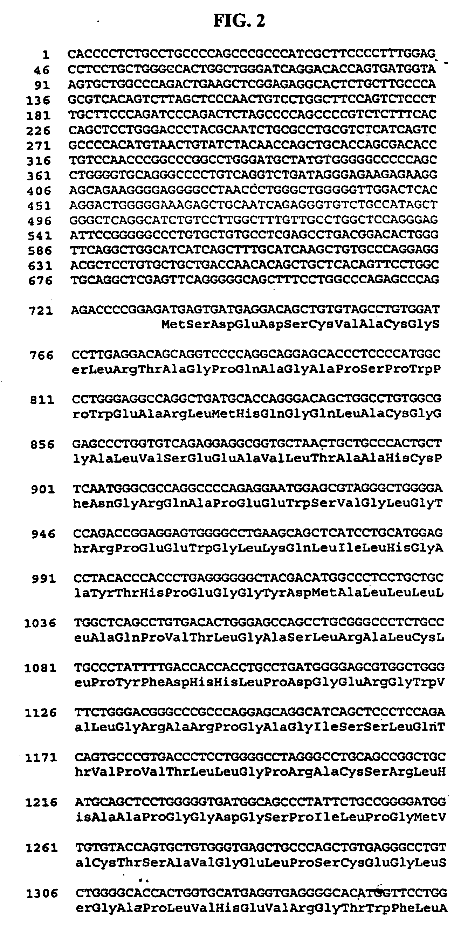

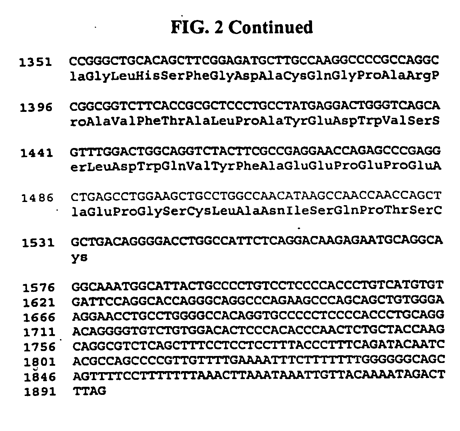

A SECP2 nucleic acid and polypeptide according to the invention includes a nucleic acid sequence (SEQ ID NO:3) and an encoded polypeptide sequence (SEQ ID NO:4) of clone 11618130.0.27. FIG. 2 illustrates the nucleic acid sequence and amino acid sequence, as well as the alignment between these two sequences.

This clone includes a nucleotide sequence (SEQ ID NO:3) of 1894 nucleotides. The nucleotide sequence includes an open reading frame (ORF) encoding a polypeptide of 267 amino acid residues with a predicted molecular weight of 28043 Daltons. The start codon is at nucleotides 732-734 and the stop codon is at nucleotides 1534-1536. The protein encoded by clone 11618130.0.27 is predicted by the PSORT program to localize in the microbody (peroxisome) with a certainty of 0.5035. The program SignalP predicts that there is no signal peptide in the encoded polypeptide.

A search of the sequence databases using BLAST P and BLASTX reveals that clone 11618130.0.27 has 330 of 333 residues (99%) identical to and positive with a 571 residue human protein termed PRO351 (PCT Publication WO9946281-A2 published September 16, 1999). In addition, it was found to have 83 of 250 residues (33%) identical to, and 119 of 250 residues (47%) positive with the 343 residue human prostasin precursor (EC 3.4.21.-) (SWISSPROT-ACC:Q16651).

The proteins of the invention encoded by clone 11618130.0.27 includes the protein disclosed as being encoded by the ORF described herein, as well as any mature protein arising therefrom as a result of post-translational modification. Thus, the protein of the invention encompasses both a precursor and any active forms of the 11618130.0.27 protein.

### SECP3

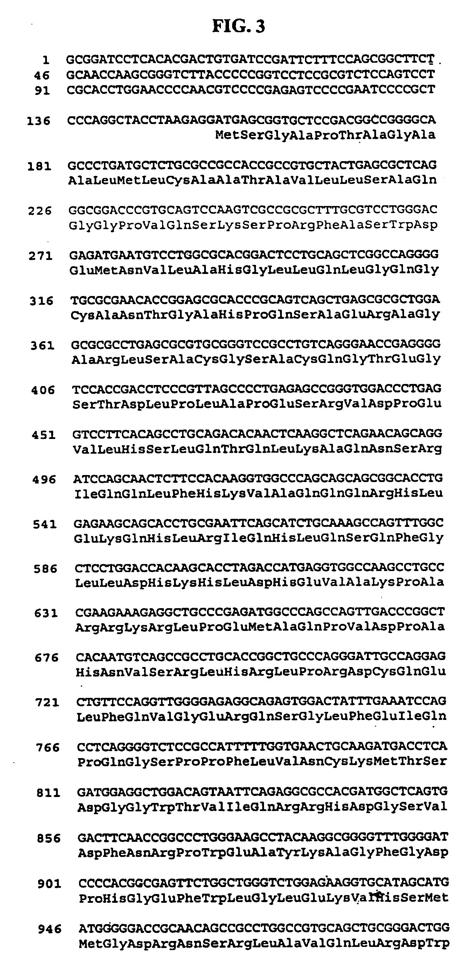

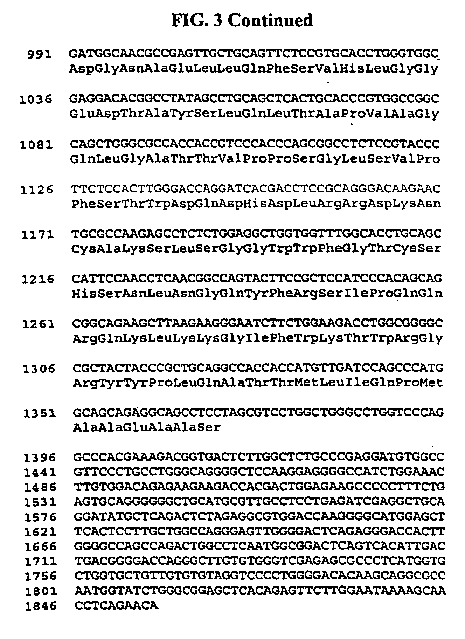

A SECP3 nucleic acid and polypeptide according to the invention includes the nucleic acid sequence (SEQ ID NO:5) and encoded polypeptide sequence (SEQ ID NO:6) of clone 11696905-0-47. FIG. 3 illustrates the nucleic acid sequence and amino acid sequence, as well as the alignment between these two sequences.

Clone 11696905-0-47 was obtained from fetal brain. In addition, RNA sequences were also found to be present in tissues including, uterus, pregnant and non-pregnant uterus, ovarian tumor, placenta, bone marrow, hippocampus, synovial membrane, fetal heart, fetal lung, pineal gland and melanocytes. This clone includes a nucleotide sequence of 1855 bp (SEQ ID NO:5). The nucleotide sequence includes an open reading frame (ORF) encoding a polypeptide of 405 amino acid residues (SEQ ID NO:6) with a predicted molecular weight of 44750 Daltons. The start codon is located at nucleotides 154-156 and the stop codon is located at nucleotides 1369-1371. The protein encoded by clone 11696905-0-47 is predicted by the PSORT program to localize extracellularly with a certainty of 0.7332. The program SignalP predicts that there is a signal peptide with the most probable cleavage site located between residues 25 and 26, in the sequence AQG-GP.

Real-time gene expression analysis was performed on SECP3 (clone 11696905-0-47). The results demonstrate that RNA sequences homologous to clone 11696905-0-47 are detected in various cell types. Cell types include adipose, adrenal gland, thyroid, brain, heart, skeletal muscle, bone marrow, colon, bladder, liver, lung, mammary gland, placenta, and testis, and in neoplastic cells derived from renal carcinoma A498, lung carcinoma NCI-H460, and melanoma SK-MEL-28.

Accordingly, SECP3 nucleic acids according to the invention can be used to identify one or more of these cell types. The presence of RNA sequences homologous to a SECP3 nucleic in a sample indicates that the sample contains one or more of the above-cell types.

A search of the sequence databases using BLASTX reveals that clone 11696905-0-47 has 403 of 405 residues (99%) identical to, and 404 of 405 residues (99%) positive with, the 405 residue human angiopoietin-related protein (SPTREMBL-ACC:Q9Y5B3). Angiopoietin homologues are useful to stimulate cell growth and tissue development. The polypeptides of clone 11696905-0-47 tend to be found as multimeric proteins (see Example 7) and are believed to have angiogenic or hematopoietic activity. They can thus be used in assays for angiogenic activity, as well as used therapeutically to stimulate restoration of vascular structure in various tissues. Examples of such uses include, but are not limited to, treatment of full-thickness skin wounds, including venous stasis ulcers and other chronic, non-healing wounds, as well as fracture repair, skin grafting, reconstructive surgery, and establishment of vascular networks in transplanted cells and tissues.

The proteins of the invention encoded by clone 11696905-0-47 include the protein disclosed as being encoded by the ORF described herein, as well as any mature protein arising therefrom as a result of post-translational modifications. Thus, the proteins of the invention encompass both a precursor and any active forms of the clone 11696905-0-47 protein.

### SECP4

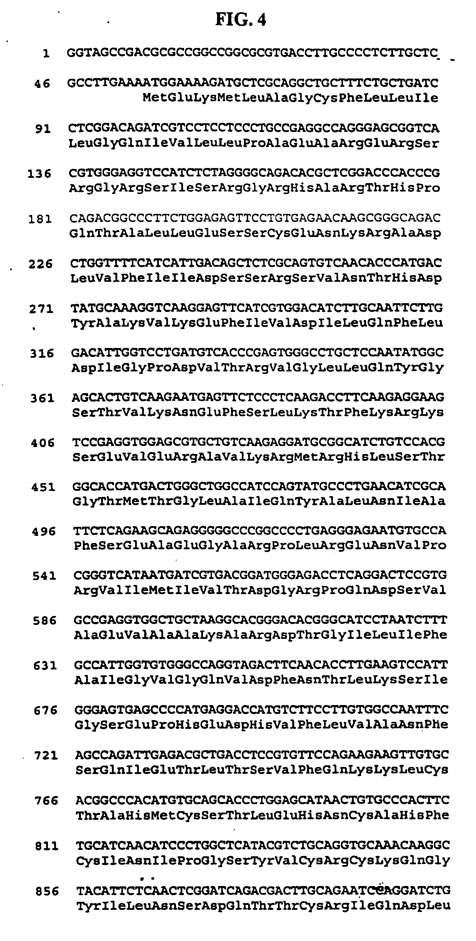

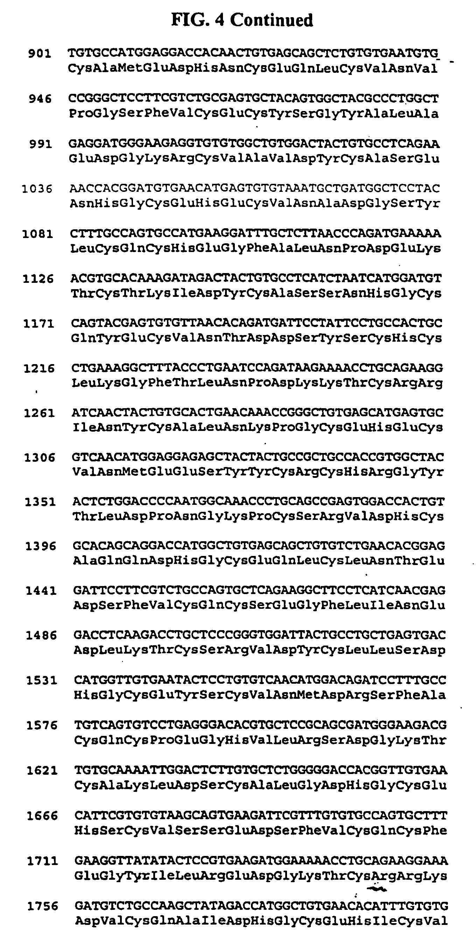

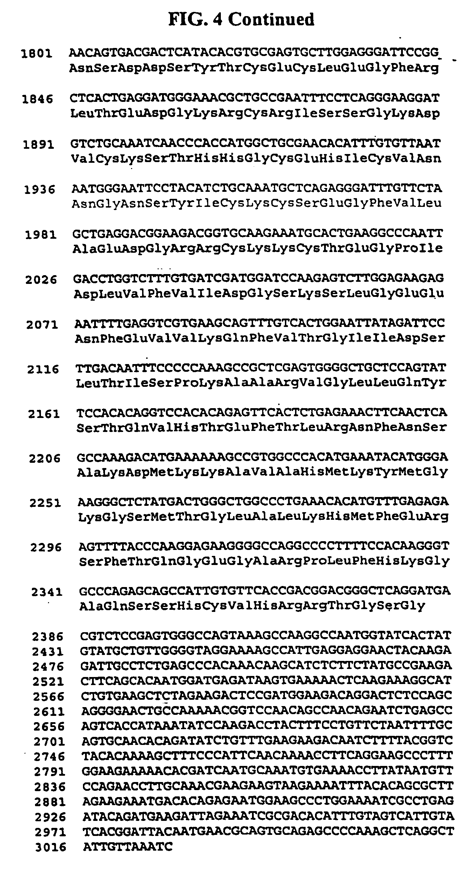

A SECP4 nucleic acid and polypeptide according to the invention includes the nucleic acid sequence (SEQ ID NO:7) and encoded polypeptide sequence (SEQ ID NO:8) of 14578444.0.143. FIG. 4 illustrates the nucleic acid sequence and amino acid sequence, as well as the alignment between these two sequences.

Clone 14578444.0.143 was obtained from fetal brain. This clone includes a nucleotide sequence (SEQ ID NO:7) of 3026 bp. The nucleotide sequence includes an open reading frame (ORF) encoding a polypeptide of 776 amino acid residues (SEQ ID NO:8) with a predicted molecular weight of 86220.8 Daltons. The start codon is located at nucleotides 55-57 and the stop codon is located at nucleotides 2384-2386. The protein encoded by clone 14578444.0.143 is predicted by the PSORT program to localize in the endoplasmic reticulum (membrane) with a certainty of 0.8200. The program SignalP predicts that there is a signal peptide with the most probable cleavage site located between residues 23 and 24 in the sequence AEA-RE.

A search of the sequence databases using BLASTX reveals that clone 14578444.0.143 has 655 of 757 residues (86%) identical to, and 702 of 757 residues (92%) positive with, the 956 residue murine matrilin-2 precursor protein (SWISSPROT-ACC:O08746), extending over residues 1-754 of the reference protein. Additional similarities are found with lower identities in residues 649-837 of the murine protein. Additionally, the search shows that there is a lower degree of similarity to murine matrilin-4 precursor. The protein of clone 14578444.0.143 also has 595 of 606 residues (98%) identical to, and 598 of 606 residues (98%) positive with, the 632 residue human matrilin-3 (PCT publication WO9904002-A1).

The matrilin proteins and polynucleotides can be used for treating a variety of developmental disorders (e.g., renal tubular acidosis, anemia, Cushing's syndrome). The proteins can serve as targets for antagonists that should be of use in treating diseases related to abnormal vesicle trafficking. These may include, but are not limited to, diseases such as cystic fibrosis, glucose-galactose malabsorption syndrome, hypercholesterolaemia, diabetes mellitus, diabetes insipidus, hyper- and hypoglycemia, Graves disease, goiter, Cushing's disease, Addison's disease, gastrointestinal disorders including ulcerative colitis, gastric and duodenal ulcers, and other conditions associated with abnormal vesicle trafficking including AIDS, and allergies including hay fever, asthma, and urticaria (hives), autoimmune hemolytic anemia, proliferative glomerulonephritis, inflammatory bowel disease, multiple sclerosis, myasthenia gravis, rheumatoid and osteoarthritis, scleroderma, Chediak-Higashi and Sjogren's syndromes, systemic lupus erythematosus, toxic shock syndrome, traumatic tissue damage, and viral, bacterial, fungal, helminth, protozoal infections, a neoplastic disorder (*e*.*g*., adenocarcinoma, leukemia, lymphoma, melanoma, myeloma, sarcoma, teratocarcinoma, and cancers), or an immune disorder, (*e*.*g*., AIDS, Addison's disease, adult respiratory distress syndrome, allergies, anemia, asthma, atherosclerosis, bronchitis, cholecystitis, Crohn's disease and ulcerative colitis).

The proteins of the invention encoded by clone 14578444.0.143 include the protein disclosed as being encoded by the ORF described herein, as well as any mature protein arising therefrom as a result of post-translational modifications. Thus, the proteins of the invention encompass both a precursor and any active forms of the proteins encoded by clone 14578444.0.143 (SECP4).

### SECP5

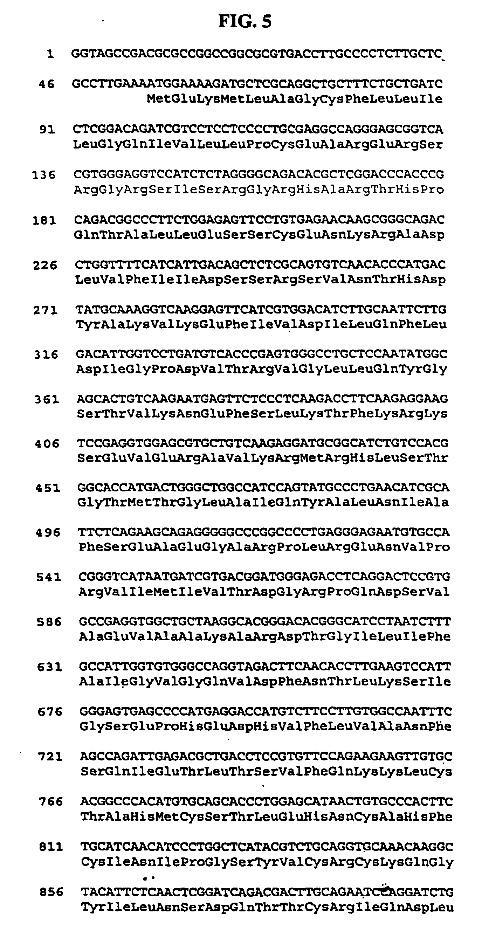

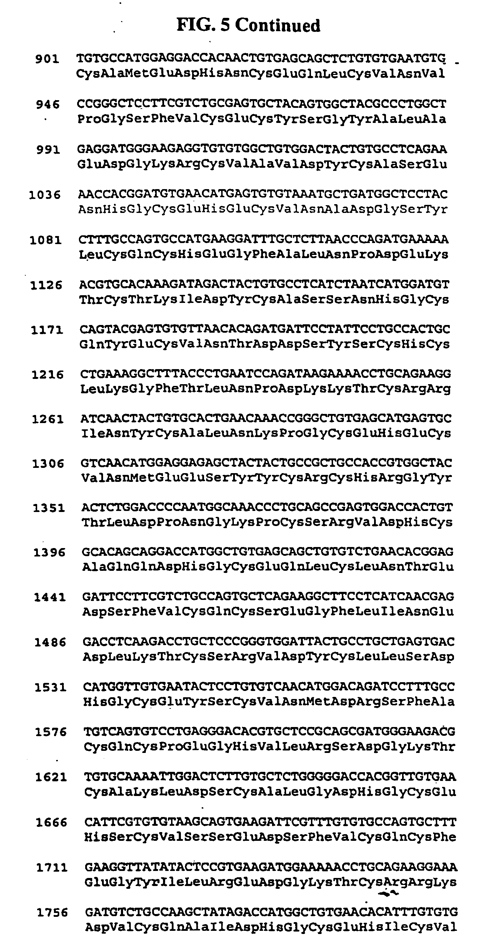

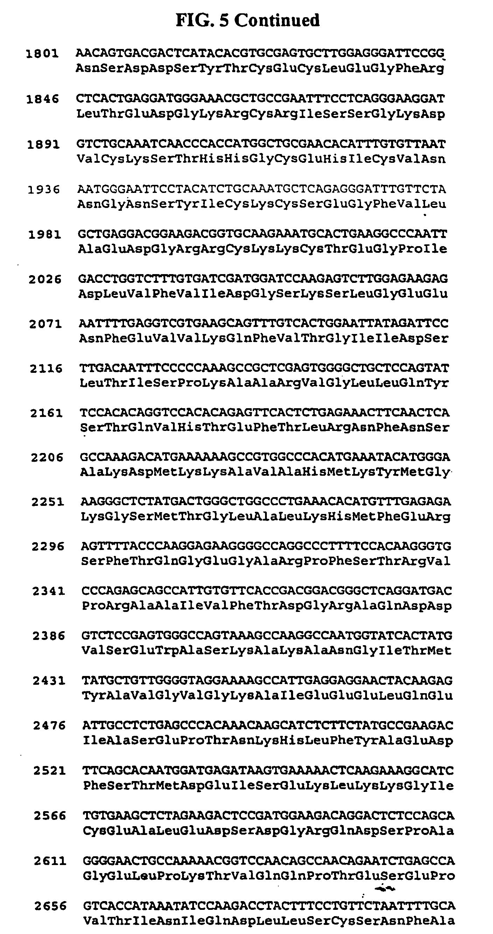

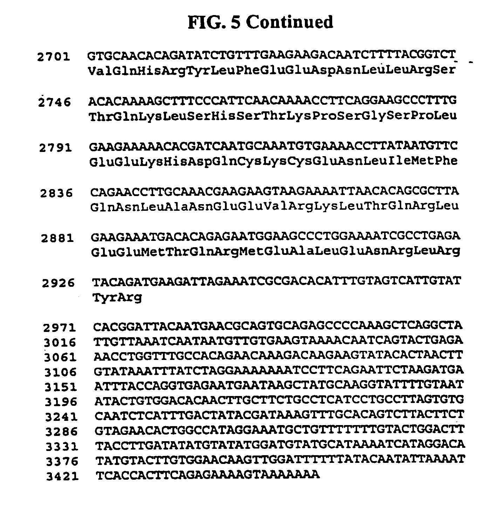

A SECP5 nucleic acid and polypeptide according to the invention includes the nucleic acid sequence (SEQ ID NO:9) and encoded polypeptide sequence (SEQ ID NO:10) of clone 14578444.0.47. FIG. 5 illustrates the nucleic acid sequence and amino acid sequence, as well as the alignment between these two sequences.

Clone 14578444.0.47 was obtained from fetal brain. This clone includes a nucleotide sequence (SEQ ID NO:9) of 3447 bp. The nucleotide sequence includes an open reading frame (ORF) encoding a polypeptide of 959 amino acid residues (SEQ ID NO:10) with a predicted molecular weight of 107144 Daltons. The start codon is located at nucleotides 55-57 and the stop codon is located at nucleotides 2933-2935. The protein encoded by clone 14578444.0.47 is predicted by the PSORT program to localize to the endoplasmic reticulum (membrane) with a certainty of 0.8200. The program SignalP predicts that there is a signal peptide with the most probable cleavage site located between residues 23 and 24 in the sequence AEA-RE.

A search of the sequence databases using BLASTX reveals that clone 14578444.0.47 has 829 of 959 residues (86%) identical to, and 887 of 959 residues (92%) positive with, the 956 residue murine matrilin-2 precursor protein (ACC: SWISSPROT-ACC:O08746). The protein encoded by clone 14578444.0.47 also has 594 of 606 residues (98%) identical to, and 597 of 606 residues (98%) positive with, the 632 residue human matrilin-3 (PCT publication WO9904002). In addition, the protein encoded by clone 14578444.0.47 also has 616 of 678 residues (90%) identical to, and 632 of 678 residues (93%) positive with the 915 residue human protein PR0219 (PCT publication WO9914328-A2).

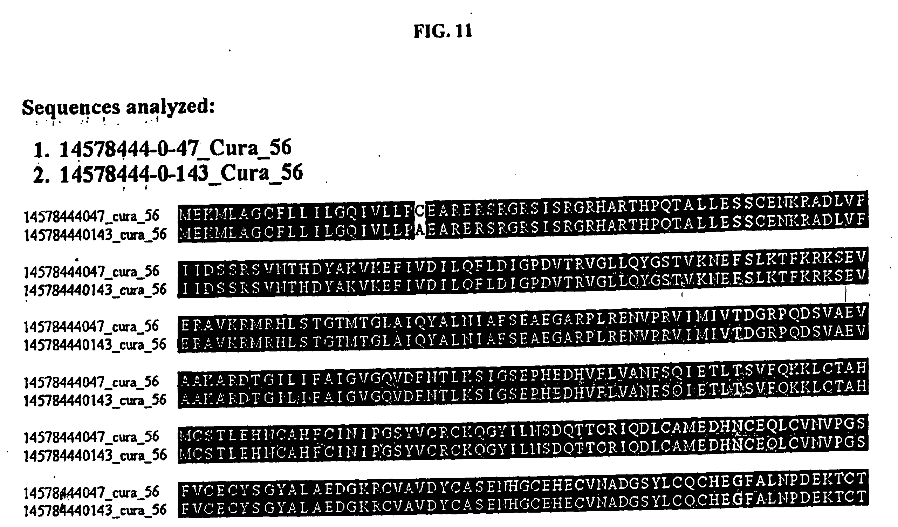

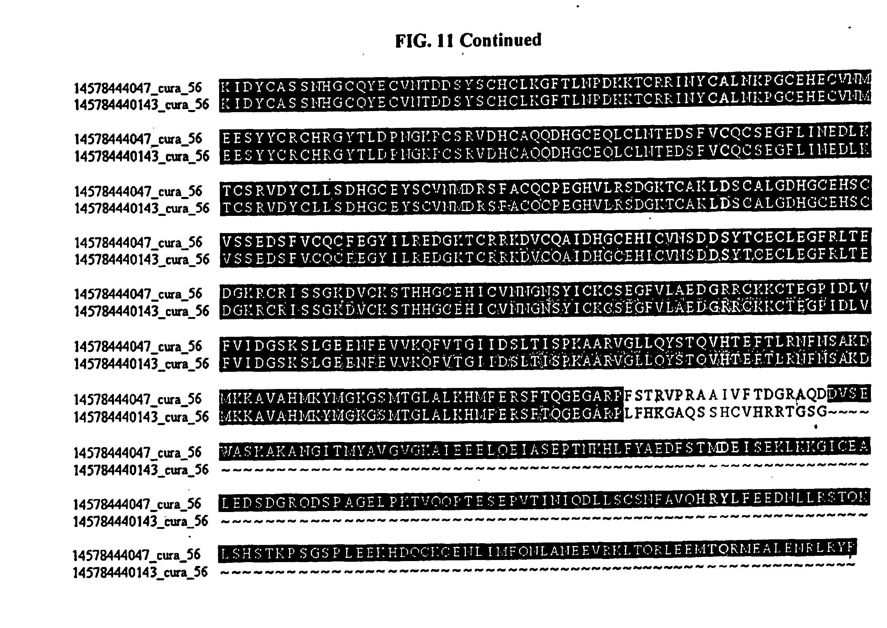

The proteins encoded by clones 14578444.0.143 (SECP4) and 14578444.0.47 (SECP5) are compared in an amino acid residue alignment shown in FIG. 11. It can be seen that the main portion of the two proteins starting with their amino-termini are virtually identical, and that short sequences in each corresponding to the carboxyl-terminal sequence of the shorter protein, clone 14578444.0.143, differ from one another. Furthermore, clone 14578444.0.47 has an extended carboxyl-terminal sequence that is missing in clone 14578444.0.143. Therefore, clones 14578444.0.143 (SECP4) and 14578444.0.47 (SECP5) are apparently related to one another as splice variants, with respect to their sequences at the carboxyl-terminal ends.

The matrilin proteins and polynucleotides can be used for treating a variety of developmental disorders (*e.g*., renal tubular acidosis, anemia, Cushing's syndrome). The proteins can serve as targets for antagonists that should be of use in treating diseases related to abnormal vesicle trafficking. These may include, but are not limited to, diseases such as cystic fibrosis, glucose-galactose malabsorption syndrome, hypercholesterolaemia, diabetes mellitus, diabetes insipidus, hyper- and hypoglycemia, Graves disease, goiter, Cushing's disease, Addison's disease, gastrointestinal disorders including ulcerative colitis, gastric and duodenal ulcers, and other conditions associated with abnormal vesicle trafficking including AIDS, and allergies including hay fever, asthma, and urticaria (hives), autoimmune hemolytic anemia, proliferative glomerulonephritis, inflammatory bowel disease, multiple sclerosis, myasthenia gravis, rheumatoid and osteoarthritis, scleroderma, Chediak-Higashi and Sjogren's syndromes, systemic lupus erythematosus, toxic shock syndrome, traumatic tissue damage, and viral, bacterial, fungal, helminth, protozoal infections, a neoplastic disorder (*e.g*., adenocarcinoma, leukemia, lymphoma, melanoma, myeloma, sarcoma, teratocarcinoma, and cancers), or an immune disorder, (*e.g.*, AIDS, Addison's disease, adult respiratory distress syndrome, allergies, anemia, asthma, atherosclerosis, bronchitis, cholecystitis, Crohn's disease and ulcerative colitis).

The proteins of the invention encoded by clone 14578444.0.47 include the protein disclosed as being encoded by the ORF described herein, as well as any mature protein arising therefrom as a result of post-translational modifications. Thus, the proteins of the invention encompass both a precursor and any active forms of the proteins encoded by clone 14578444.0.47 (SECP5).

### SECP6

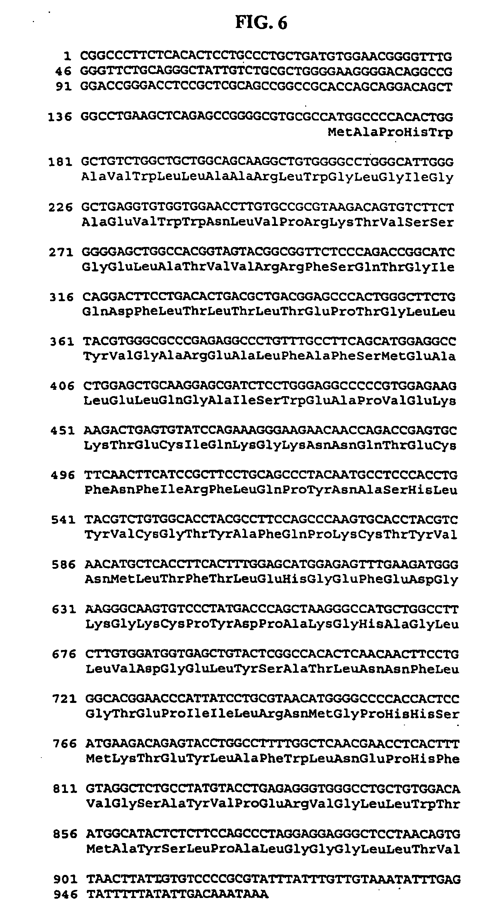

A SECP6 nucleic acid and polypeptide according to the invention includes the nucleic acid sequence (SEQ ID NO:11) and encoded polypeptide sequence (SEQ ID NO:12) of clone 14998905.0.65. FIG. 6 illustrates the nucleic acid sequence and amino acid sequence, as well as the alignment between these two sequences.

Clone 14998905.0.65 was obtained from lymphoid tissue, in particular, from the lymph node. This clone includes a nucleotide sequence (SEQ ID NO:11) of 967 bp. The nucleotide sequence includes an open reading frame (ORF) encoding a polypeptide of 245 amino acid residues (SEQ ID NO:12) with a predicted molecular weight of 27327.2 Daltons. The start codon is located at nucleotides 166-168 and the stop codon is located at nucleotides 902-904. The protein encoded by clone 14998905.0.65 is predicted by the PSORT program to localize in the microbody (peroxisome) with a certainty of 0.7480. PSORT predicts that there is no amino-terminal signal sequence. Conversely, the program SignalP predicts that there is a signal peptide with the most probable cleavage site located between residues 20 and 21, in the sequence GIG-AE.

A search of the sequence databases using BLASTX reveals that clone 14998905.0.65 has 204 of 226 residues (90%) identical to, and 214 of 226 residues (94%) positive with, the 834 residue murine semaphorin 4C precursor protein (SWISSPROT-ACC:Q64151). Semaphorin 4C is indicated as being a Type I membrane protein widely expressed in the nervous system during development. In addition, it contains one immunoglobulin-like C2-type domain. The protein encoded by clone 14998905.0.65 also has similarities to mouse CD100 antigen (PCT publication WO9717368-A1) and to human semaphorin (JP10155490-A).

The proteins of the invention encoded by clone 14998905.0.65 include the protein disclosed as being encoded by the ORF described herein, as well as any mature protein arising therefrom as a result of post-translational modifications. Thus, the proteins of the invention encompass both a precursor and any active forms of the clone 14998905.0.65 protein.

### SECP7

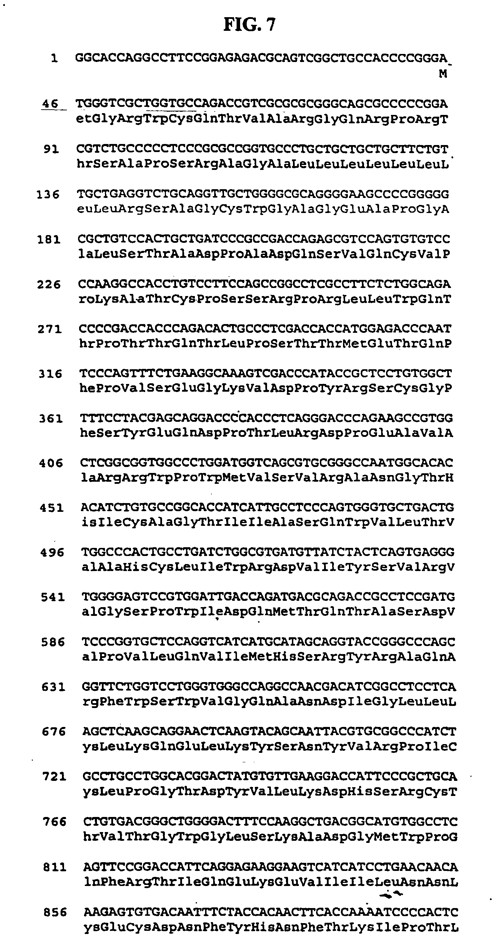

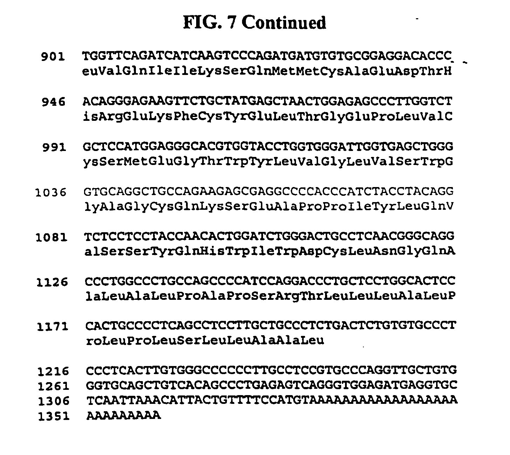

A SECP7 nucleic acid and polypeptide according to the invention includes the nucleic acid sequence (SEQ ID NO:13) and encoded polypeptide sequence (SEQ ID NO:14) of clone 16406477.0.206. FIG. 7 illustrates the nucleic acid sequence and amino acid sequence, as well as the alignment between these two sequences.

Clone 16406477.0.206 was obtained from testis. In addition, sequences of clone 16406477.0.206 were also found in an RNA pool derived from adrenal gland, mammary gland, prostate gland, testis, uterus, bone marrow, melanoma, pituitary gland, thyroid gland and spleen. This clone includes a nucleotide sequence (SEQ ID NO:13) comprising of 1359 bp with an open reading frame (ORF) encoding a polypeptide of 385 amino acid residues (SEQ ID NO:14) with a predicted molecular weight of 43087.3 Daltons. The start codon is located at nucleotides 45-47 and the stop codon is located at nucleotides 1201-1203. The protein encoded by clone 16406477.0.206 is predicted by the PSORT program to localize extracellularly with a certainty of 0.5804 and to have a cleavable amino-terminal signal sequence. The program SignalP predicts that there is a signal peptide with the most probable cleavage site located between residues 39 and 40, in the sequence CWG-AG.

Real-time expression analysis was performed on SECP7 (clone 16406477.0.206). The results demonstrate that RNA homologous to this clone is found in multiple cell and tissue types. These cells and tissues include brain, mammary gland, and testis, and in neoplastic cells derived from ovarian carcinoma OVCAR-3, ovarian carcinoma OVCAR-5, ovarian carcinoma OVCAR-8, ovarian carcinoma IGROV-1, breast carcinoma (pleural effusion) T47D, breast carcinoma BT-549, melanoma M14. Real-time gene expression analysis was performed on SECP3 (clone 11696905-0-47). The results demonstrate that RNA sequences homologous to clone 11696905-0-47 are detected in various cell types. Cell types include adipose, adrenal gland, thyroid, brain, heart, skeletal muscle, bone marrow, colon, bladder, liver, lung, mammary gland, placenta, and testis, and in neoplastic cells derived from renal carcinoma A498, lung carcinoma NCI-H460, and melanoma SK-MEL-28.

Accordingly, SECP7 nucleic acids according to the invention can be used to identify one or more of these cell types. The presence of RNA sequences homologous to a SECP7 nucleic in a sample indicates that the sample contains one or more of the above-cell types.

A search of the sequence databases using BLASTX reveals that clone 16406477.0.206 is 100% identical to a human testis-specific protein TSP50 (SPTREMBL-ACC:Q9UI38) with a trypsin/chymotrypsin-like domain. In addition, the protein encoded by clone 16406477.0.206 has low similarity to the 343 residue human prostasin precursor (EC 3.4.21.-) (SWISSPROT ACC:Q16651).

The proteins of the invention encoded by clone 16406477.0.206 include the protein disclosed as being encoded by the ORF described herein, as well as any mature protein arising therefrom as a result of post-translational modifications. Thus, the proteins of the invention encompass both a precursor and any active forms of the clone 16406477.0.206 protein.

### SECP8

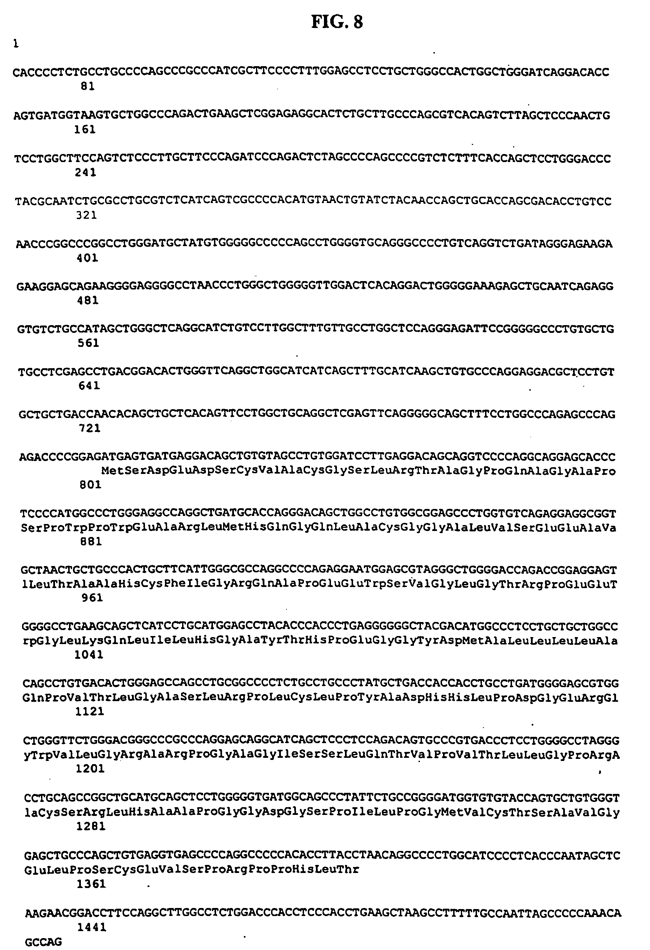

A SECP8 nucleic acid and polypeptide according to the invention includes the nucleic acid sequence (SEQ ID NO:15) and encoded polypeptide sequence (SEQ ID NO:16) of clone 11618130.0.184. FIG. 8 illustrates the nucleic acid sequence and amino acid sequence, as well as the alignment between these two sequences.

Clone 11618130.0.184 includes a nucleotide sequence (SEQ ID NO:15) of 1445 bp. The nucleotide sequence includes an open reading frame (ORF) encoding a polypeptide of 198 amino acid residues (SEQ ID NO:16) with a predicted molecular weight of 20659 Daltons. The start codon is located at nucleotides 732-734 and the stop codon is located at nucleotides 1326-1328. The protein encoded by clone 11618130.0.184 is predicted by the PSORT program to localize in the cytoplasm. The program SignalP predicts that there is no signal peptide.

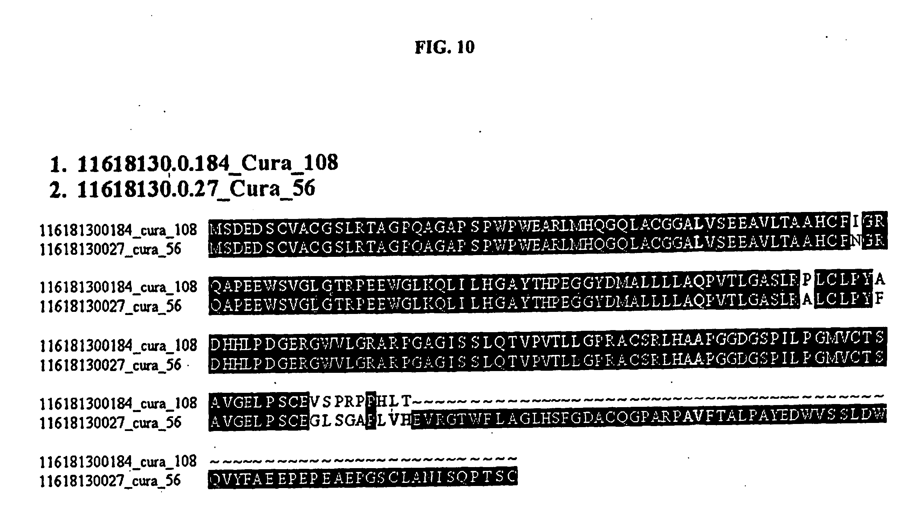

Clones 11618130.0.184 (SECP8) and 11618130.0.27 (SECP2) resemble each other in that they are identical over most of their common sequences, and differ only at the carboxyl-terminal end. In addition, clone 11618130.0.27 extends further at the carboxyl-terminal end than does clone 11618130.0.184. An alignment of clones 11618130.0.27 and 11618130.0.184 is shown in FIG. 10.

The proteins of the invention encoded by clone 11618130.0.184 include the protein disclosed as being encoded by the ORF described herein, as well as any mature protein arising therefrom as a result of post-translational modifications. Thus, the proteins of the invention encompass both a precursor and any active forms of the 11618130.0.184 protein.

### SECP9

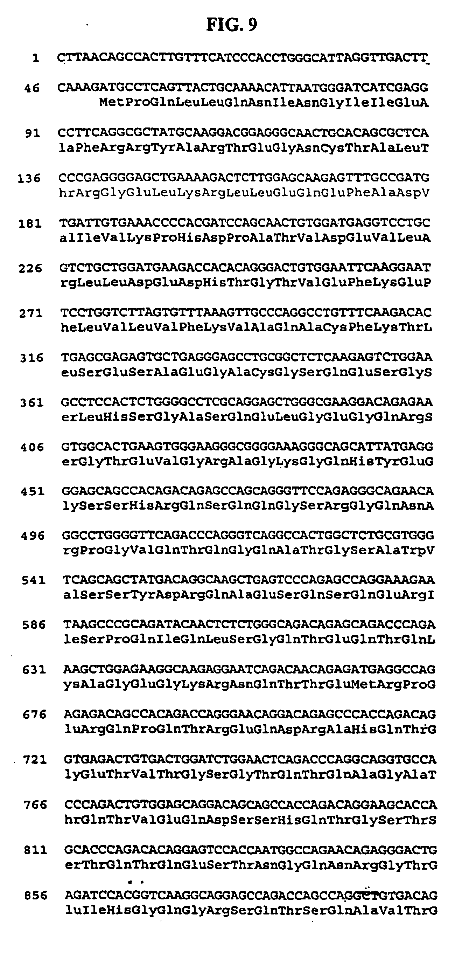

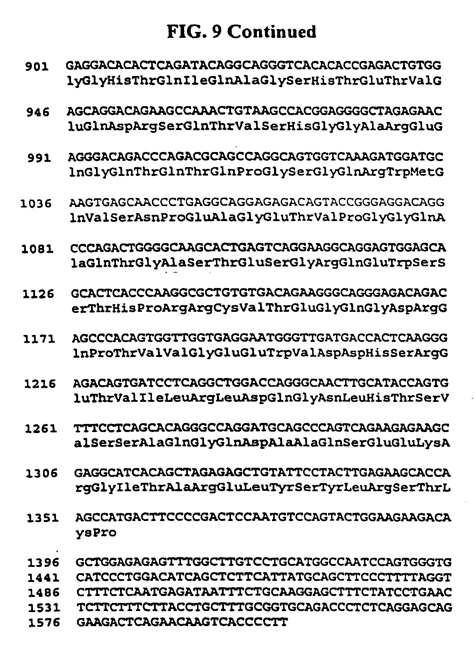

A SECP9 nucleic acid and polypeptide according to the invention includes the nucleic acid sequence (SEQ ID NO:17) and encoded polypeptide sequence (SEQ ID NO:18) of clone 21637262.0.64. FIG. 9 illustrates the nucleic acid sequence and amino acid sequence, as well as the alignment between these two sequences.

Clone 21637262.0.64 was obtained from salivary gland. This clone includes a nucleotide sequence (SEQ ID NO:17) of 1600 bp. The nucleotide sequence includes an open reading frame (ORF) encoding a polypeptide of 435 amino acid residues (SEQ ID NO:18) with a predicted molecular weight of 47162.5 Daltons. The start codon is located at nucleotides 51-53 and the stop codon is located at nucleotides 1356-1358. The protein encoded by clone 21637262.0.64 is predicted by the PSORT program to localize in the cytoplasm with a certainty of 0.4500. The program PSORT and program SignalP predict that the protein appears to have no amino-terminal signal sequence.

Real-time expression analysis was performed on SECP9 (clone 21637262.0.64). The results demonstrate that RNA homologous to this clone is present in multiple tissue and cell types. The relative amounts of RNA in various cell types are shown in FIG. 14 (see also the Examples, below). The cells include myometrium, placenta, uterus, prostate, and testis, and neoplastic cells derived from breast carcinoma (pleural effusion) T47D, breast carcinoma (pleural effusion) MDA-MB-231, breast carcinoma BT-549, ovarian carcinoma OVCAR-3, ovarian carcinoma OVCAR-5, prostate carcinoma (bone metastases) PC-3, melanoma M14, and melanoma LOX IMVI.

Accordingly, SECP9 nucleic acids according to the invention can be used to identify one or more of these cell types. The presence of RNA sequences homologous to a SECP9 nucleic in a sample indicates that the sample contains one or more of the above-cell types.

A search of the sequence databases using BLASTX reveals that clone 21637262.0.64 has 23 of 420 residues (29%) identical to, and 201 of 420 residues (47%) positive with, the 1130 residue murine protein repetin (SWISSPROT-ACC:P97347). Repetin is a member of the "fused gene" subgroup within the S100 gene family that is an epidermal differentiation protein.

The proteins of the invention encoded by clone 21637262.0.64 include the protein disclosed as being encoded by the ORF described herein, as well as any mature protein arising therefrom as a result of post-translational modifications. Thus, the proteins of the invention encompass both a precursor and any active forms of the clone 21637262.0.64 protein.

### SECP Nucleic Acids

The novel nucleic acids of the invention include those that encode a SECP or SECP-like protein, or biologically-active portions thereof. The nucleic acids include nucleic acids encoding polypeptides that include the amino acid sequence of one or more of SEQ ID NO:1, 3, 5, 7, 9, 11, 13, 15, and/or 17. The encoded polypeptides can thus include, *e*.*g*., the amino acid sequences of SEQ ID NO: 2, 4, 6, 8, 10, 12, 14, 16, and/or 18.

In some embodiments, a SECP polypeptide or protein, as disclosed herein, includes the product of a naturally-occurring polypeptide, precursor form, pro-protein, or mature form of the polypeptide. The naturally-occurring polypeptide, precursor, or pro-protein includes, e.g., the full-length gene product, encoded by the corresponding gene. The naturally-occurring polypeptide also includes the polypeptide, precursor or pro-protein encoded by an open reading frame (ORF) described herein. As used herein, the term "idcntical" residues corresponds to those residues in a comparison between two sequences where the equivalent nucleotide base or amino acid residue in an alignment of two sequences is the same residue. Residues are alternatively described as "similar" or "positive" when the comparisons between two sequences in an alignment show that residues in an equivalent position in a comparison are either the same amino acid residue or a conserved amino acid residue, as defined below.

As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full length gene product, encoded by the corresponding gene. Alternatively, it may be defined as the polypeptide, precursor or proprotein encoded by an open reading frame described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the amino-terminal methionine residue encoded by the initiation codon of an open reading frame, or the proteolytic cleavage of a signal peptide or leader sequence. Thus, a mature form arising from a precursor polypeptide or protein that has residues 1 to N, where residue 1 is the amino-terminal methionine, would have residues 2 through N remaining after removal of the amino-terminal methionine. Alternatively, a mature form arising from a precursor polypeptide or protein having residues 1 to N, in which an amino-terminal signal sequence from residue 1 to residue M is cleaved, would have the residues from residue M+1 to residue N remaining. Further, as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

In some embodiments, a nucleic acid encoding a polypeptide having the amino acid sequence of one or more of SEQ ID NO:2, 4, 6, 8, 10, 12, 14, 16, and/or 18, includes the nucleic acid sequence of any of SEQ ID NO:1, 3, 5, 7, 9, 11, 13, 15, and/or 17, or a fragment thereof. Additionally, the invention includes mutant or variant nucleic acids of any of SEQ ID NO:1, 3, 5, 7, 9, 11, 13, 15, and/or 17, or a fragment thereof, any of whose bases may be changed from the disclosed sequence while still encoding a protein that maintains its SECP-like biological activities and physiological functions. The invention further includes the complement of the nucleic acid sequence of any of SEQ ID NO:1, 3, 5, 7, 9, 11, 13, 15, and/or 17, including fragments, derivatives, analogs and homologs thereof. The invention additionally includes nucleic acids or nucleic acid fragments, or complements thereto, whose structures include chemical modifications.

Also included are nucleic acid fragments sufficient for use as hybridization probes to identify SECP-encoding nucleic acids (*e*.*g*., SECP mRNA) and fragments for use as polymerase chain reaction (PCR) primers for the amplification or mutation of SECP nucleic acid molecules. As used herein, the term "nucleic acid molecule" is intended to include DNA molecules (*e*.*g*., cDNA or genomic DNA), RNA molecules (*e*.*g*., mRNA), analogs of the DNA or RNA generated using nucleotide analogs, and derivatives, fragments, and homologs thereof. The nucleic acid molecule can be single-stranded or double-stranded, but preferably is double-stranded DNA.

The term "probes" refer to nucleic acid sequences of variable length, preferably between at least about 10 nucleotides (nt), 100 nt, or as many as about, *e*.*g*., 6,000 nt, depending upon the specific use. Probes are used in the detection of identical, similar, or complementary nucleic acid sequences. Longer length probes are usually obtained from a natural or recombinant source, are highly specific and much slower to hybridize than oligomers. Probes may be single- or double-stranded, and may also be designed to have specificity in PCR, membrane-based hybridization technologies, or ELISA-like technologies.

The tem "isolated" nucleic acid molecule is a nucleic acid that is separated from other nucleic acid molecules that are present in the natural source of the nucleic acid. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules. Preferably, an "isolated" nucleic acid is free of sequences which naturally flank the nucleic acid (i.e., sequences located at the 5'- and 3'-termini of the nucleic acid) in the genomic DNA of the organism from which the nucleic acid is derived. For example, in various embodiments, the isolated SECP nucleic acid molecule can contain less than aprroximately 50 kb, 25 kb, 5 kb,
4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e*.*g*., a nucleic acid molecule having the nucleotide sequence of SEQ ID NO:1, 3, 5, 7, 9, 11, 13, 15, and/or 17, or a complement of any of these nucleotide sequences, can be isolated using standard molecular biology techniques and the sequence information provided herein. Using all or a portion of the nucleic acid sequence of any of SEQ ID NO:1, 3, 5, 7, 9, 11, 13, 15, and/or 17 as a hybridization probe, SECP nucleic acid sequences can be isolated using standard hybridization and cloning techniques (*e*.*g*., as described in Sambrook *et al*., eds., MOLECULAR CLONING: A LABORATORY MANUAL 2^{nd} Ed., Cold Spring Harbor Laboratory Press, Cold Spring Harbor, NY, 1989; and Ausubel, *et al.,* eds., CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to SECP nucleotide sequences can be prepared by standard synthetic techniques, *e*.*g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment, an oligonucleotide comprising a nucleic acid molecule less than 100 nt in length would further comprise at lease 6 contiguous nucleotides of any of SEQ ID NO:1, 3, 5, 7, 9, 11, 13, 15, and/or 17, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in any of SEQ ID NO:1, 3, 5, 7, 9, 11, 13, 15, and/or 17. In still another embodiment, an isolated nucleic acid molecule of the invention comprises a nucleic acid molecule that is a complement of the nucleotide sequence shown in any of SEQ ID NO:1, 3, 5, 7, 9, 11, 13, 15, and/or 17, or a portion of this nucleotide sequence. A nucleic acid molecule that is complementary to the nucleotide sequence shown in is one that is sufficiently complementary to the nucleotide sequence shown in of any of SEQ ID NO:1, 3, 5, 7, 9, 11, 13, 15, and/or 17, that it can hydrogen bond with little or no mismatches to the nucleotide sequence shown in of any of SEQ ID NO:1, 3, 5, 7, 9, 11, 13, 15, and/or 17, thereby forming a stable duplex.

As used herein, the term "complementary" refers to Watson-Crick or Hoogsteen base-pairing between nucleotides units of a nucleic acid molecule, whereas the term "binding" is defined as the physical or chemical interaction between two polypeptides or compounds or associated polypeptides or compounds or combinations thereof. Binding includes ionic, non-ionic, Von der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Additionally, the nucleic acid molecule of the invention can comprise only a portion of the nucleic acid sequence of any of SEQ ID NO:1, 3, 5, 7, 9, 11, 13, 15, and/or 17, *e*.*g*., a fragment that can be used as a probe or primer, or a fragment encoding a biologically active portion of SECP. Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild-type.

Derivatives and analogs may be full-length or other than full-length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, 85%, 90%, 95%, 98%, or even 99% identity (with a preferred identity of 80-99%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g*. Ausubel, *et al.,* CURRENT PROTOCOLS IN MOLECULAR BIOLOGY, John Wiley & Sons, New York, NY, 1993, and below. An exemplary program is the Gap program (Wisconsin Sequence Analysis Package, Version 8 for UNIX, Genetics Computer Group, University Research Park, Madison, WI) using the default settings, which uses the algorithm of Smith and Waterman (Adv. Appl. Math., 1981, 2: 482-489), which is incorporated herein by reference in its entirety.

The tern "homologous nucleic acid sequence" or "homologous amino acid sequence," or variations thereof, refer to sequences characterized by a homology at the nucleotide level or amino acid level as previously discussed. Homologous nucleotide sequences encode those sequences coding for isoforms of SECP polypeptide. Isoforms can be expressed in different tissues of the same organism as a result of, *e*.*g*., alternative splicing of RNA. Alternatively, isoforms can be encoded by different genes. In the invention, homologous nucleotide sequences include nucleotide sequences encoding for a SECP polypeptide of species other than humans, including, but not limited to, mammals, and thus can include, *e*.*g*., mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the nucleotide sequence encoding human SECP protein. Homologous nucleic acid sequences include those nucleic acid sequences that encode conservative amino acid substitutions (see below) in any of SEQ ID NO:1, 3, 5, 7, 9, 11, 13, 15, and/or 17, as well as a polypeptide having SECP activity. Biological activities of the SECP proteins are described below. A homologous amino acid sequence does not encode the amino acid sequence of a human SECP polypeptide.

The nucleotide sequence determined from the cloning of the human SECP gene allows for the generation of probes and primers designed for use in identifying the cell types disclosed and/or cloning SECP homologues in other cell types, *e*.*g*., from other tissues, as well as SECP homologues from other mammals. The probe/primer typically comprises a substantially-purified oligonucleotide. The oligonucleotide typically comprises a region of nucleotide sequence that hybridizes under stringent conditions to at least about 12, 25, 50, 100, 150, 200, 250, 300, 350 or 400 or more consecutive sense strand nucleotide sequence of SEQ ID NO:1, 3, 5, 7, 9, 11, 13, 15, and/or 17; or an anti-sense strand nucleotide sequence of SEQ ID NO:1, 3, 5, 7, 9, 11, 13, 15, and/or 17; or of a naturally occurring mutant of SEQ ID NO:1, 3, 5, 7, 9, 11, 13, 15, and/or 17.

Probes based upon the human SECP nucleotide sequence can be used to detect transcripts or genomic sequences encoding the same or homologous proteins. In various embodiments, the probe further comprises a label group attached thereto, e.g., the label group can be a radioisotope, a fluorescent compound, an enzyme, or an enzyme co-factor. Such probes can be used as a part of a diagnostic test kit for identifying cells or tissue which mis-express a SECP protein, such as by measuring a level of a SECP-encoding nucleic acid in a sample of cells from a subject *e*.*g*., detecting SECP mRNA levels or determining whether a genomic SECP gene has been mutated or deleted.

The term "a polypeptide having a biologically-active portion of SECP" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of SECP" can be prepared by isolating a portion of SEQ ID NO:1, 3, 5, 7, 9, 11, 13, 15, and/or 17, that encodes a polypeptide having a SECP biological activity, expressing the encoded portion of SECP protein (*e*.*g*., by recombinant expression *in vitro*), and assessing the activity of the encoded portion of SECP.

### SECP Variants

The invention further encompasses nucleic acid molecules that differ from the disclosed SECP nucleotide sequences due to degeneracy of the genetic code. These nucleic acids therefore encode the same SECP protein as those encoded by the nucleotide sequence shown in SEQ ID NO:1, 3, 5, 7, 9, 11, 13, 15, and/or 17. In another embodiment, an isolated nucleic acid molecule of the invention has a nucleotide sequence encoding a protein having an amino acid sequence shown in any of SEQ ID NO:1, 3, 5, 7, 9, 11, 13, 15, and/or 17.

In addition to the human SECP nucleotide sequence shown in any of SEQ ID NO:1, 3, 5, 7, 9, 11, 13, 15, and/or 17, it will be appreciated by those skilled in the art that DNA sequence polymorphisms that lead to changes in the amino acid sequences of SECP may exist within a population (*e*.*g*., the human population). Such genetic polymorphism in the SECP gene may exist among individuals within a population due to natural allelic variation. As used herein, the terms "gene" and "recombinant gene" refer to nucleic acid molecules comprising an open reading frame encoding a SECP protein, preferably a mammalian SECP protein. Such natural allelic variations can typically result in 1-5% variance in the nucleotide sequence of the SECP gene. Any and all such nucleotide variations and resulting amino acid polymorphisms in SECP that are the result of natural allelic variation and that do not alter the functional activity of SECP are intended to be within the scope of the invention.

Additionally, nucleic acid molecules encoding SECP proteins from other species, and thus that have a nucleotide sequence that differs from the human sequence of any of SEQ ID NO:1, 3, 5, 7, 9, 11, 13, 15, and/or 17, are intended to be within the scope of the invention. Nucleic acid molecules corresponding to natural allelic variants and homologues of the SECP cDNAs of the invention can be isolated based on their homology to the human SECP nucleic acids disclosed herein using the human cDNAs, or a portion thereof, as a hybridization probe according to standard hybridization techniques under stringent hybridization conditions.

In another embodiment, an isolated nucleic acid molecule of the invention is at least 6 nucleotides in length and hybridizes under stringent conditions to the nucleic acid molecule comprising the nucleotide sequence of any of SEQ ID NO:1, 3, 5, 7, 9, 11, 13, 15, and/or 17. In another embodiment, the nucleic acid is at least 10, 25, 50, 100, 250, 500 or 750 nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i*.*e*., nucleic acids encoding SECP proteins derived from species other than human) or other related sequences (*e*.*g*., paralogs) can be obtained by low, moderate or high stringency hybridization with all or a portion of the particular human sequence as a probe using methods well known in the art for nucleic acid hybridization and cloning.

As used herein; the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tₘ) for the specific sequence at a defined ionic strength and pH. The Tₘ is the temperature (under defined ionic strength, pH and nucleic acid concentration) at which 50% of the probes complementary to the target sequence hybridize to the target sequence at equilibrium. Since the target sequences are generally present at excess, at Tₘ, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e*.*g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions is hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C. This hybridization is followed by one or more washes in 0.2X SSC, 0.01% BSA at 50°C. An isolated nucleic acid molecule of the invention that hybridizes under stringent conditions to the sequence of any of SEQ ID NO:1, 3, 5, 7, 9, 11, 13, 15, and/or 17 corresponds to a naturally occurring nucleic acid molecule. As used herein, a "naturally-occurring" nucleic acid molecule refers to an RNA or DNA molecule having a nucleotide sequence that occurs in nature (*e*.*g*., encodes a natural protein).

In a second embodiment, a nucleic acid sequence that is hybridizable to the nucleic acid molecule comprising the nucleotide sequence of any of SEQ ID NO:1, 3, 5, 7, 9, 11, 13, 15, and/or 17, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well known in the art. See, *e*.*g*., Ausubel *et al*. (eds.), 1993, CURRENT PROTOCOLS IN MOLECULAR BIOLOGY, John Wiley & Sons, NY, and Kriegler, 1990. GENE TRANSFER AND EXPRESSION, A LABORATORY MANUAL, Stockton Press, NY.

In a third embodiment, a nucleic acid that is hybridizable to the nucleic acid molecule comprising the nucleotide sequence of any of SEQ ID NO:1, 3, 5, 7, 9, 11, 13, 15, and/or 17, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e*.*g*., as employed for cross-species hybridizations). *See*, *e*.*g*., Ausubel, *et al*., (eds.), 1993. CURRENT PROTOCOLS IN MOLECULAR BIOLOGY, John Wiley & Sons, NY, and Kriegler, 1990. GENE TRANSFER AND EXPRESSION, A LABORATORY MANUAL, Stockton Press, NY; Shilo and Weinberg, 1981. *Proc. Natl*. *Acad. Sci. USA 78:* 6789-6792.

### Conservative Mutations

In addition to naturally-occurring allelic variants of the SECP sequence that may exist in the population, the skilled artisan will further appreciate that changes can be introduced by mutation into the nucleotide sequence of any of SEQ ID NO:1, 3, 5, 7, 9, 11, 13, 15, and/or 17, thereby leading to changes in the amino acid sequence of the encoded SECP protein, without altering the functional ability of the SECP protein. For example, nucleotide substitutions leading to amino acid substitutions at "non-essential" amino acid residues can be made in the sequence of any of SEQ ID NO:1, 3, 5, 7, 9, 11, 13, 15, and/or 17. A "non-essential" amino acid residue is a residue that can be altered from the wild-type sequence of SECP without altering the biological activity, whereas an "essential" amino acid residue is required for biological activity. For example, amino acid residues that are conserved among the SECP proteins of the invention, are predicted to be particularly non-amenable to such alteration.

Amino acid residues that are conserved among members of a SECP family members are predicted to be less amenable to alteration. For example, a SECP protein according to the invention can contain at least one domain that is a typically conserved region in a SECP family member. As such, these conserved domains are not likely to be amenable to mutation. Other amino acid residues, however, (*e*.*g*., those that are not conserved or only semi-conserved among members of the SECP family) may not be as essential for activity and thus are more likely to be amenable to alteration.

Another aspect of the invention pertains to nucleic acid molecules encoding SECP proteins that contain changes in amino acid residues that are not essential for activity. Such SECP proteins differ in amino acid sequence from any of any of SEQ ID NO:2, 4, 6, 8, 10, 12, 14, 16, and/or 18, yet retain biological activity. In one embodiment, the isolated nucleic acid molecule comprises a nucleotide sequence encoding a protein, wherein the protein comprises an amino acid sequence at least about 75% homologous to the amino acid sequence of any of SEQ ID NO:2, 4, 6, 8, 10, 12, 14, 16, and/or 18. Preferably, the protein encoded by the nucleic acid is at least about 80% homologous to any of SEQ ID NO:2, 4, 6, 8, 10, 12, 14, 16, and/or 18, more preferably at least about 90%, 95%, 98%, and most preferably at least about 99% homologous to SEQ ID NO:2, 4, 6, 8, 10, 12, 14, 16, and/or 18.

An isolated nucleic acid molecule encoding a SECP protein homologous to the protein of any of SEQ ID NO:2, 4, 6, 8, 10, 12, 14, 16, and/or 18 can be created by introducing one or more nucleotide substitutions, additions or deletions into the corresponding nucleotide sequence (*i*.*e*., SEQ ID NO:1, 3, 5, 7, 9, 11, 13, 15, and/or 17), such that one or more amino acid substitutions, additions or deletions are introduced into the encoded protein.

Mutations can be introduced into SEQ ID NO:1, 3, 5, 7, 9, 11, 13, 15, and/or 17 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e*.*g*., lysine, arginine, histidine), acidic side chains (*e*.*g*., aspartic acid, glutamic acid), uncharged polar side chains (*e*.*g*., glycine, asparagine, glutamine, serine, threonine, tyrosine, cysteine), nonpolar side chains (*e*.*g*., alanine, valine, leucine, isoleucine, proline, phenylalanine, methionine, tryptophan), β-branched side chains (*e*.*g*., threonine, valine, isoleucine) and aromatic side chains (*e*.*g*., tyrosine, phenylalanine, tryptophan, histidine). Thus, a predicted nonessential amino acid residue in SECP is replaced with another amino acid residue from the same side chain family. Alternatively, in another embodiment, mutations can be introduced randomly along all or part of a SECP coding sequence, such as by saturation mutagenesis, and the resultant mutants can be screened for SECP biological activity to identify mutants that retain activity. Following mutagenesis of SEQ ID NO:1, 3, 5, 7, 9, 11, 13, 15, and/or 17, the encoded protein can be expressed by any recombinant technology known in the art and the activity of the protein can be determined.

In one embodiment, a mutant SECP protein can be assayed for: (*i*) the ability to form protein:protein interactions with other SECP proteins, other cell-surface proteins, or biologically-active portions thereof; (*ii*) complex formation between a mutant SECP protein and a SECP receptor; (*iii*) the ability of a mutant SECP protein to bind to an intracellular target protein or biologically active portion thereof; (*e*.*g*., avidin proteins); (*iv*) the ability to bind BRA protein; or (*v*) the ability to specifically bind an anti-SECP protein antibody.

### Antisense Nucleic Acids

Another aspect of the invention pertains to isolated antisense nucleic acid molecules that are hybridizable to or complementary to the nucleic acid molecule comprising the nucleotide sequence of SEQ ID NO:1, 3, 5, 7, 9, 11, 13, 15, and/or 17, or fragments, analogs or derivatives thereof. An "antisense" nucleic acid comprises a nucleotide sequence that is complementary to a "sense" nucleic acid encoding a protein, e.g., complementary to the coding strand of a double-stranded cDNA molecule or complementary to an mRNA sequence. In specific aspects, antisense nucleic acid molecules are provided that comprise a sequence complementary to at least about 10, 25, 50, 100, 250 or 500 nucleotides or an entire SECP coding strand, or to only a portion thereof. Nucleic acid molecules encoding fragments, homologs, derivatives and analogs of a SECP protein of any of SEQ ID NO:2, 4, 6, 8, 10, 12, 14, 16, and/or 18 or antisense nucleic acids complementary to a SECP nucleic acid sequence of SEQ ID NO:2, 4, 6, 8, 10, 12, 14, 16, and/or 18, are additionally provided.

In one embodiment, an antisense nucleic acid molecule is antisense to a "coding region" of the coding strand of a nucleotide sequence encoding SECP. The term "coding region" refers to the region of the nucleotide sequence comprising codons which are translated into amino acid residues (*e*.*g*., the protein coding region of a human SECP that corresponds to any of SEQ ID NO:2, 4, 6, 8, 10, 12, 14, 16, and/or 18. In another embodiment, the antisense nucleic acid molecule is antisense to a "non-coding region" of the coding strand of a nucleotide sequence encoding SECP. The term "non-coding region" refers to 5'- and 3'-terminal sequences which flank the coding region that are not translated into amino acids (*i*.*e*., also referred to as 5' and 3' non-translated regions).

Given the coding strand sequences encoding the SECP proteins disclosed herein (*e*.*g*., SEQ ID NO:1, 3, 5, 7, 9, 11, 13, 15, and/or 17), antisense nucleic acids of the invention can be designed according to the rules of Watson and Crick or Hoogsteen base-pairing. The antisense nucleic acid molecule can be complementary to the entire coding region of SECP mRNA, but more preferably is an oligonucleotide that is antisense to only a portion of the coding or non-coding region of SECP mRNA. For example, the antisense oligonucleotide can be complementary to the region surrounding the translation start site of SECP mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e*.*g*., an antisense oligonucleotide) can be chemically synthesized using naturally-occurring nucleotides or variously modified nucleotides designed to increase the biological stability of the molecules or to increase the physical stability of the duplex formed between the antisense and sense nucleic acids, *e*.*g*., phosphorothioate derivatives and acridine-substituted nucleotides can be used.

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a SECP protein to thereby inhibit expression of the protein, e.g., by inhibiting transcription and/or translation. The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e*.*g*., by linking the antisense nucleic acid molecules to peptides or antibodies that bind to cell surface receptors or antigens). The antisense nucleic acid molecules can also be delivered to cells using the vectors described herein. To achieve sufficient intracellular concentrations of antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual α-units, the strands run parallel to each other (*see,* Gaultier, *et al*., 1987. *Nucl. Acids Res.* 15: 6625-6641). The antisense nucleic acid molecule can also comprise a 2'-o-methylribonucleotide (Inoue, *et al*., 1987. *Nucl. Acids Res.* 15: 6131-6148) or a chimeric RNA-DNA analogue (Inoue, *et al.,* 1987. *FEBS Lett.* 215: 327-330).

### Ribozymes and PNA Moieties

Such modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In still another embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e*.*g*., hammerhead ribozymes; described by Haselhoff and Gerlach, 1988. *Nature* 334: 585-591) can be used to catalytically-cleave SECP mRNA transcripts to thereby inhibit translation of SECP mRNA. A ribozyme having specificity for a SECP-encoding nucleic acid can be designed based upon the nucleotide sequence of a SECP DNA disclosed herein (*i*.*e*., SEQ ID NO:1, 3, 5, 7, 9, 11, 13, 15, and/or 17). For example, a derivative of a *Tetrahymena* L-19 IVS RNA can be constructed in which the nucleotide sequence of the active site is complementary to the nucleotide sequence to be cleaved in a SECP-encoding mRNA. See, *e*.*g*., Cech, *et al*., U.S. Patent No. 4,987,071; and Cech, *et al.,* U.S. Patent No. 5,116,742. Alternatively, SECP mRNA can be used to select a catalytic RNA having a specific ribonuclease activity from a pool of RNA molecules (Bartel, *et al*., 1993. *Science* 261: 1411-1418).

Alternatively, SECP gene expression can be inhibited by targeting nucleotide sequences complementary to the regulatory region of the SECP (*e*.*g*., the SECP promoter and/or enhancers) to form triple helical structures that prevent transcription of the SECP gene in target cells. *See, e*.*g*., Helene, 1991. *Anticancer Drug Des.* 6: 569-84; Helene, *et al.,* 1992. *Ann. N*.*Y*. *Acad*. *Sci.* 660: 27-36; and Maher, 1992. *Bioassays* 14: 807-15.

In various embodiments, the nucleic acids of SECP can be modified at the base moiety, sugar moiety or phosphate backbone to improve, *e*.*g*., the stability, hybridization, or solubility of the molecule. For example, the deoxyribose phosphate backbone of the nucleic acids can be modified to generate peptide nucleic acids (Hyrup, *et al.,* 1996. *Bioorg. Med. Chem.* 4: 5-23). As used herein, the terms "peptide nucleic acids" or "PNAs" refer to nucleic acid mimics, *e*.*g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup, *et al*., 1996. *supra;* Perry-O'Keefe, *et al*., 1996. *Proc. Natl*. *Acad. Sci. USA* 93: 14670-14675.

PNAs of SECP can be used in therapeutic and diagnostic applications. For example, PNAs can be used as antisense or antigene agents for sequence-specific modulation of gene expression by, *e*.*g*., inducing transcription or translation arrest or inhibiting replication. PNAs of SECP can also be used, *e*.*g*., in the analysis of single base pair mutations in a gene by, *e.g.,* PNA directed PCR clamping; as artificial restriction enzymes when used in combination with other enzymes, *e*.*g*., S1 nucleases (*see,* Hyrup, 1996., *supra*); or as probes or primers for DNA sequence and hybridization (*see,* Hyrup, *et al.,* 1996.; Perry-O'Keefe, 1996., *supra*).

In another embodiment, PNAs of SECP can be modified, *e*.*g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of SECP can be generated that may combine the advantageous properties of PNA and DNA. Such chimeras allow DNA recognition enzymes, *e.g*., RNase H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (*see,* Hyrup, 1996., *supra*). The synthesis of PNA-DNA chimeras can be performed as described in Finn, *et al*., (1996. *Nucl. Acids Res.* 24: 3357-3363). For example, a DNA chain can be synthesized on a solid support using standard phosphoramidite coupling chemistry, and modified nucleoside analogs, *e*.*g*., 5'-(4-methoxytrityl)amino-5'-deoxy-thymidine phosphoramidite, can be used between the PNA and the 5' end of DNA (Mag, *et al*., 1989. *Nucl. Acid Res.* 17: 5973-5988). PNA monomers are then coupled in a stepwise manner to produce a chimeric molecule with a 5' PNA segment and a 3' DNA segment (*see*, Finn, *et al*., 1996., *supra*). Alternatively, chimeric molecules can be synthesized with a 5' DNA segment and a 3' PNA segment. *See, e*.*g*., Petersen, *et al*., 1975. *Bioorg*. *Med*. *Chem. Lett.* 5: 1119-11124.

In other embodiments, the oligonucleotide may include other appended groups such as peptides (*e*.*g*., for targeting host cell receptors *in vivo*), or agents facilitating transport across the cell membrane (*see, e*.*g*., Letsinger, *et al.,* 1989. *Proc. Natl. Acad. Sci. U*.*S*.*A*. 86: 6553-6556; Lemaitre, *et al*., 1987. *Proc. Natl. Acad. Sci*. 84: 648-652; PCT Publication No. WO88/09810) or the blood-brain barrier (*see, e.g.,* PCT Publication No. WO 89/10134). In addition, oligonucleotides can be modified with hybridization triggered cleavage agents (*see, e.g.,* Krol, *et al*., 1988. *BioTechniques* 6:958-976) or intercalating agents *(see, e.g.,* Zon, 1988. *Pharm. Res.* 5: 539-549). To this end, the oligonucleotide may be conjugated to another molecule, *e*.*g*., a peptide, a hybridization triggered cross-linking agent, a transport agent, a hybridization-triggered cleavage agent, and the like.

### Characterization of SECP Polypeptides

A polypeptide according to the invention includes a polypeptide including the amino acid sequence of SECP polypeptides whose sequences are provided in SEQ ID NO:2, 4, 6, 8, 10, 12, 14, 16, and/or 18. The invention also includes a mutant or variant protein any of whose residues may be changed from the corresponding residues shown in SEQ ID NO:2, 4, 6, 8, 10, 12, 14, 16, and/or 18, while still encoding a protein that maintains its SECP activities and physiological functions, or a functional fragment thereof.

In general, a SECP variant that preserves SECP-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated SECP proteins, and biologically-active portions thereof, or derivatives, fragments, analogs or homologs thereof. Also provided are polypeptide fragments suitable for use as immunogens to raise anti-SECP antibodies. In one embodiment, native SECP proteins can be isolated from cells or tissue sources by an appropriate purification scheme using standard protein purification techniques. In another embodiment, SECP proteins are produced by recombinant DNA techniques. Alternative to recombinant expression, a SECP protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the SECP protein is derived, or substantially free from chemical precursors or other chemicals when chemically synthesized. The language "substantially free of cellular material" includes preparations of SECP proteins in which the protein is separated from cellular components of the cells from which it is isolated or recombinantly-produced. In one embodiment, the language "substantially free of cellular material" includes preparations of SECP proteins having less than about 30% (by dry weight) ofnon-SECP proteins (also referred to herein as a "contaminating protein"), more preferably less than about 20% of non-SECP proteins, still more preferably less than about 10% of non-SECP proteins, and most preferably less than about 5% of non-SECP proteins. When the SECP protein or biologically-active portion thereof is recombinantly-produced, it is also preferably substantially free of culture medium, *i*.*e*., culture medium represents less than about 20%, more preferably less than about 10%, and most preferably less than about 5% of the volume of the SECP protein preparation.

The phrase "substantially free of chemical precursors or other chemicals" includes preparations of SECP protein in which the protein is separated from chemical precursors or other chemicals that are involved in the synthesis of the protein. In one embodiment, the language "substantially free of chemical precursors or other chemicals" includes preparations of SECP protein having less than about 30% (by dry weight) of chemical precursors or non-SECP chemicals, more preferably less than about 20% chemical precursors or non-SECP chemicals, still more preferably less than about 10% chemical precursors or non-SECP chemicals, and most preferably less than about 5% chemical precursors or non-SECP chemicals.

Biologically-active portions of a SECP protein include peptides comprising amino acid sequences sufficiently homologous to or derived from the amino acid sequence of the SECP protein which include fewer amino acids than the full-length SECP proteins, and exhibit at least one activity of a SECP protein. Typically, biologically-active portions comprise a domain or motif with at least one activity of the SECP protein. A biologically-active portion of a SECP protein can be a polypeptide which is, for example, 10, 25, 50, 100 or more amino acids in length.

A biologically-active portion of a SECP protein of the invention may contain at least one of the above-identified conserved domains. Moreover, other biologically active portions, in which other regions of the protein are deleted, can be prepared by recombinant techniques and evaluated for one or more of the functional activities of a native SECP protein.

In an embodiment, the SECP protein has an amino acid sequence shown in any of SEQ ID NO:1, 3, 5, 7, 9, 11, 13, 15, and/or 17. In other embodiments, the SECP protein is substantially homologous to any of SEQ ID NO:1, 3, 5, 7, 9, 11, 13, 15, and/or 17, and retains the functional activity of the protein of any of SEQ ID NO:1, 3, 5, 7, 9, 11, 13, 15, and/or 17, yet differs in amino acid sequence due to natural allelic variation or mutagenesis, as described in detail below. Accordingly, in another embodiment, the SECP protein is a protein that comprises an amino acid sequence at least about 45% homologous, and more preferably about 55, 65, 70, 75, 80, 85, 90, 95, 98 or even 99% homologous to the amino acid sequence of any of SEQ ID NO:1, 3, 5, 7, 9, 11, 13, 15, and/or 17 and retains the functional activity of the SECP proteins of the corresponding polypeptide having the sequence of SEQ ID NO:1, 3, 5, 7, 9, 11, 13, 15, and/or 17.

### Determining Homology Between Two or More Sequences

To determine the percent homology of two amino acid sequences or of two nucleic acids, the sequences are aligned for optimal comparison purposes (*e*.*g*., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. *J*. *Mol*. *Biol*. 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NO:1, 3, 5, 7, 9, 11, 13, 15, and/or 17.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e*.*g*., A, T, C, G, U, or I, in the case of nucleic acids) occurs in both sequences to yield the number of matched positions, dividing the number of matched positions by the total number of positions in the region of comparison (*i*.*e*., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides SECP chimeric or fusion proteins. As used herein, a SECP "chimeric protein" or "fusion protein" comprises a SECP polypeptide operatively-linked to a non-SECP polypeptide. An "SECP polypeptide" refers to a polypeptide having an amino acid sequence corresponding to a SECP protein shown in SEQ ID NO:2, 4, 6, 8, 10, 12, 14, 16, and/or 18, whereas a "non-SECP polypeptide" refers to a polypeptide having an amino acid sequence corresponding to a protein that is not substantially homologous to the SECP protein (*e*.*g*., a protein that is different from the SECP protein and that is derived from the same or a different organism). Within a SECP fusion protein the SECP polypeptide can correspond to all or a portion of a SECP protein. In one embodiment, a SECP fusion protein comprises at least one biologically-active portion of a SECP protein. In another embodiment, a SECP fusion protein comprises at least two biologically-active portions of a SECP protein. In yet another embodiment, a SECP fusion protein comprises at least three biologically-active portions of a SECP protein. Within the fusion protein, the term "operatively-linked" is intended to indicate that the SECP polypeptide and the non-SECP polypeptide are fused in-frame with one another. The non-SECP polypeptide can be fused to the amino-terminus or carboxyl-terminus of the SECP polypeptide.

In one embodiment, the fusion protein is a GST-SECP fusion protein in which the SECP sequences are fused to the carboxyl-terminus of the GST (glutathione S-transferase) sequences. Such fusion proteins can facilitate the purification of recombinant SECP polypeptides.

In another embodiment, the fusion protein is a SECP protein containing a heterologous signal sequence at its amino-terminus. In certain host cells (e.g., mammalian host cells), expression and/or secretion of SECP can be increased through use of a heterologous signal sequence.

In yet another embodiment, the fusion protein is a SECP-immunoglobulin fusion protein in which the SECP sequences are fused to sequences derived from a member of the immunoglobulin protein family. The SECP-immunoglobulin fusion proteins of the invention can be incorporated into pharmaceutical compositions and administered to a subject to inhibit an interaction between a SECP ligand and a SECP protein on the surface of a cell, to thereby suppress SECP-mediated signal transduction *in vivo.* The SECP-immunoglobulin fusion proteins can be used to affect the bioavailability of a SECP cognate ligand. Inhibition of the SECP ligand/SECP interaction may be useful therapeutically for both the treatment of proliferative and differentiative disorders, as well as modulating (*e*.*g*:, promoting or inhibiting) cell survival. Moreover, the SECP-immunoglobulin fusion proteins of the invention can be used as immunogens to produce anti-SECP antibodies in a subject, to purify SECP ligands, and in screening assays to identify molecules that inhibit the interaction of SECP with a SECP ligand.

A SECP chimeric or fusion protein of the invention can be produced by standard recombinant DNA techniques. For example, DNA fragments coding for the different polypeptide sequences are ligated together in-frame in accordance with conventional techniques, *e*.*g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (*see, e*.*g*., Ausubel, *et al.* (eds.) CURRENT PROTOCOLS IN MOLECULAR BIOLOGY, John Wiley & Sons, 1992). Moreover, many expression vectors are commercially available that already encode a fusion moiety (*e*.*g*., a GST polypeptide). A SECP-encoding nucleic acid can be cloned into such an expression vector such that the fusion moiety is linked in-frame to the SECP protein.

### SECP Agonists and Antagonists

The invention also pertains to variants of the SECP proteins that function as either SECP agonists (*i*.*e*., mimetics) or as SECP antagonists. Variants of the SECP protein can be generated by mutagenesis (*e*.*g*., discrete point mutation or truncation of the SECP protein). An agonist of a SECP protein can retain substantially the same, or a subset of, the biological activities of the naturally-occurring form of a SECP protein. An antagonist of a SECP protein can inhibit one or more of the activities of the naturally occurring form of a SECP protein by, for example, competitively binding to a downstream or upstream member of a cellular signaling cascade which includes the SECP protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the SECP proteins.

Variants of the SECP proteins that function as either SECP agonists (*i*.*e*., mimetics) or as SECP antagonists can be identified by screening combinatorial libraries of mutants (*e*.*g*., truncation mutants) of the SECP proteins for SECP protein agonist or antagonist activity. In one embodiment, a variegated library of SECP variants is generated by combinatorial mutagenesis at the nucleic acid level and is encoded by a variegated gene library. A variegated library of SECP variants can be produced by, for example, enzymatically-ligating a mixture of synthetic oligonucleotides into gene sequences such that a degenerate set of potential SECP sequences is expressible as individual polypeptides, or alternatively, as a set of larger fusion proteins (*e*.*g*., for phage display) containing the set of SECP sequences therein. There are a variety of methods which can be used to produce libraries of potential SECP variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential SECP sequences. Methods for synthesizing degenerate oligonucleotides are well-known within the art. *See, e*.*g*., Narang, 1983. *Tetrahedron* 39: 3; Itakura, *et al.,* 1984. *Annu. Rev. Biochem. 53:* 323; Itakura, *et al*., 1984. *Science* 198: 1056; Ike, *et al*., 1983. *Nucl*. *Acids Res.* 11: 477.

### Polypeptide Libraries

In addition, libraries of fragments of the SECP protein coding sequences can be used to generate a variegated population of SECP fragments for screening and subsequent selection of variants of a SECP protein. In one embodiment, a library of coding sequence fragments can be generated by treating a double-stranded PCR fragment of a SECP coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes amino-terminal and internal fragments of various sizes of the SECP proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of SECP proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify SECP variants. *See, e*.*g*., Arkin and Yourvan, 1992. *Proc. Natl*. *Acad. Sci. USA* 89: 7811-7815; Delgrave, *et al.,* 1993. *Protein Engineering* 6:327-331.

### Anti-SECP Antibodies

The invention encompasses antibodies and antibody fragments, such as F_{ab} or (F_{ab})₂, that bind immunospecifically to any of the SECP polypeptides of said invention.

An isolated SECP protein, or a portion or fragment thereof, can be used as an immunogen to generate antibodies that bind to SECP polypeptides using standard techniques for polyclonal and monoclonal antibody preparation. The full-length SECP proteins can be used or, alternatively, the invention provides antigenic peptide fragments of SECP proteins for use as immunogens. The antigenic SECP peptides comprises at least 4 amino acid residues of the amino acid sequence shown in SEQ ID NO:2, 4, 6, 8, 10, 12, 14, 16, and/or 18, and encompasses an epitope of SECP such that an antibody raised against the peptide forms a specific immune complex with SECP. Preferably, the antigenic peptide comprises at least 6, 8, 10, 15, 20, or 30 amino acid residues. Longer antigenic peptides are sometimes preferable over shorter antigenic peptides, depending on use and according to methods well known to someone skilled in the art.

In certain embodiments of the invention, at least one epitope encompassed by the antigenic peptide is a region of SECP that is located on the surface of the protein (*e.g*., a hydrophilic region). As a means for targeting antibody production, hydropathy plots showing regions of hydrophilicity and hydrophobicity may be generated by any method well known in the art, including, for example, the Kyte-Doolittle or the Hopp-Woods methods, either with or without Fourier transformation *(see, e.g.,* Hopp and Woods, 1981. *Proc. Nat*. *Acad. Sci. USA* 78: 3824-3828; Kyte and Doolittle, 1982. *J*. *Mol*. *Biol.* 157: 105-142, each incorporated herein by reference in their entirety).

As disclosed herein, SECP protein sequences of SEQ ID NO:2, 4, 6, 8, 10, 12, 14, 16, and/or 18, or derivatives, fragments, analogs, or homologs thereof, may be utilized as immunogens in the generation of antibodies that immunospecifically-bind these protein components. The term "antibody" as used herein refers to immunoglobulin molecules and immunologically-active portions of immunoglobulin molecules, *i*.*e*., molecules that contain an antigen binding site that specifically-binds (immunoreacts with) an antigen, such as SECP. Such antibodies include, but are not limited to, polyclonal, monoclonal, chimeric, single chain, F_{ab} and F_{(ab')2} fragments, and an F_{ab} expression library. In a specific embodiment, antibodies to human SECP proteins are disclosed. Various procedures known within the art may be used for the production of polyclonal or monoclonal antibodies to a SECP protein sequence of SEQ ID NO:2, 4, 6, 8, 10, 12, 14, 16, and/or 18, or a derivative, fragment, analog, or homolog thereof.

For the production of polyclonal antibodies, various suitable host animals (*e.g*., rabbit, goat, mouse or other mammal) may be immunized by injection with the native protein, or a synthetic variant thereof, or a derivative of the foregoing. An appropriate immunogenic preparation can contain, for example, recombinantly-expressed SECP protein or a chemically-synthesized SECP polypeptide. The preparation can further include an adjuvant. Various adjuvants used to increase the immunological response include, but are not limited to, Freund's (complete and incomplete), mineral gels (*e.g*., aluminum hydroxide), surface active substances (*e*.*g*., lysolecithin, pluronic polyols, polyanions, peptides, oil emulsions, dinitrophenol, etc.), human adjuvants such as *Bacille Calmette-Guerin* and *Corynebacterium parvum,* or similar immunostimulatory agents. If desired, the antibody molecules directed against SECP can be isolated from the mammal (*e.g*., from the blood) and further purified by well known techniques, such as protein A chromatography to obtain the IgG fraction.

The term "monoclonal antibody" or "monoclonal antibody composition", as used herein, refers to a population of antibody molecules that contain only one species of an antigen binding site capable of immunoreacting with a particular epitope of SECP. A monoclonal antibody composition thus typically displays a single binding affinity for a particular SECP protein with which it immunoreacts. For preparation of monoclonal antibodies directed towards a particular SECP protein, or derivatives, fragments, analogs or homologs thereof, any technique that provides for the production of antibody molecules by continuous cell line culture may be utilized. Such techniques include, but are not limited to, the hybridoma technique (*see, e*.*g*., Kohler & Milstein, 1975. *Nature* 256: 495-497); the trioma technique; the human B-cell hybridoma technique (*see, e*.*g*., Kozbor, *et al*., 1983. *Immunol*. *Today* 4: 72) and the EBV hybridoma technique to produce human monoclonal antibodies (*see, e.g.,* Cole, *et al*., 1985. In: MONOCLONAL ANTIBODIES AND CANCER THERAPY, Alan R. Liss, Inc., pp. 77-96). Human monoclonal antibodies may be utilized in the practice of the invention and may be produced by using human hybridomas *(see, e*.*g*., Cote, *et al*., 1983. *Proc Natl Acad Sci USA* 80: 2026-2030) or by transforming human B-cells with Epstein Barr Virus *in vitro* (*see, e.g.,* Cole, *et al*., 1985. In: MONOCLONAL ANTIBODIES AND CANCER THERAPY, Alan R. Liss, Inc., pp. 77-96). Each of the above citations is incorporated herein by reference in their entirety.

According to the invention, techniques can be adapted for the production of single-chain antibodies specific to a SECP protein *(see, e*.*g*., U.S. Patent No. 4,946,778). In addition, methods can be adapted for the construction of F_{ab} expression libraries *(see, e*.*g*., Huse, *et al*., 1989. *Science* 246: 1275-1281) to allow rapid and effective identification of monoclonal F_{ab} fragments with the desired specificity for a SECP protein or derivatives, fragments, analogs or homologs thereof. Non-human antibodies can be "humanized" by techniques well known in the art. *See, e.g.,* U.S. Patent No. 5,225,539. Antibody fragments that contain the idiotypes to a SECP protein may be produced by techniques known in the art including, but not limited to:
(*i*) an F_{(ab')2} fragment produced by pepsin digestion of an antibody molecule; (*ii*) an F_{ab} fragment generated by reducing the disulfide bridges of an F_{(ab')2} fragment; (*iii*) an F_{ab} fragment generated by the treatment of the antibody molecule with papain and a reducing agent and (*iv*) Fᵥ fragments.

Additionally, recombinant anti-SECP antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in International Application No. PCT/US86/02269; European Patent Application No. 184,187; European Patent Application No. 171,496; European Patent Application No. 173,494; PCT International Publication No. WO 86/01533; U.S. Patent No. 4,816,567; U.S. Pat. No. 5,225,539; European Patent Application No. 125,023; Better, *et al*., 1988. *Science* 240: 1041-1043; Liu, *et al.,* 1987. *Proc. Natl. Acad. Sci. USA* 84: 3439-3443; Liu, *et al.,* 1987. *J*. *Immunol*. 139: 3521-3526; Sun, *et al*., 1987. *Proc. Natl*. *Acad. Sci. USA* 84: 214-218; Nishimura, *et al*., 1987. *Cancer Res. 47:* 999-1005; Wood, *et al*., 1985. *Nature* 314 :446-449; Shaw, *et al*., 1988. *J*. *Natl. Cancer Inst.* 80: 1553-1559); Morrison(1985) *Science* 229:1202-1207; Oi, *et al.* (1986) *BioTechniques* 4:214; Jones, *et al*., 1986. *Nature* 321: 552-525; Verhoeyan, *et al*., 1988. *Science* 239: 1534; and Beidler, *et al*., 1988. *J*. *Immunol*. 141: 4053-4060. Each of the above citations are incorporated herein by reference in their entirety.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of a SECP protein is facilitated by generation of hybridomas that bind to the fragment of a SECP protein possessing such a domain. Thus, antibodies that are specific for a desired domain within a SECP protein, or derivatives, fragments, analogs or homologs thereof, are also provided herein.

Anti-SECP antibodies may be used in methods known within the art relating to the localization and/or quantitation of a SECP protein (*e.g.,* for use in measuring levels of the SECP protein within appropriate physiological samples, for use in diagnostic methods, for use in imaging the protein, and the like). In a given embodiment, antibodies for SECP proteins, or derivatives, fragments, analogs or homologs thereof, that contain the antibody derived binding domain, are utilized as pharmacologically-active compounds (hereinafter "Therapeutics").

An anti-SECP antibody (*e*.*g*., monoclonal antibody) can be used to isolate a SECP polypeptide by standard techniques, such as affinity chromatography or immunoprecipitation. An anti-SECP antibody can facilitate the purification of natural SECP polypeptide from cells and of recombinantly-produced SECP polypeptide expressed in host cells. Moreover, an anti-SECP antibody can be used to detect SECP protein (e.g., in a cellular lysate or cell supernatant) in order to evaluate the abundance and pattern of expression of the SECP protein. Anti-SECP antibodies can be used diagnostically to monitor protein levels in tissue as part of a clinical testing procedure, *e*.*g*., to, for example, determine the efficacy of a given treatment regimen. Detection can be facilitated by coupling (*i*.*e*., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### SECP Recombinant Expression Vectors and Host Cells

Another aspect of the invention pertains to vectors, preferably expression vectors, containing a nucleic acid encoding a SECP protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present Specification, "plasmid" and "vector" can be used interchangeably, as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e*.*g*., in an *in vitro* transcription/translation system or in a host cell when the vector is introduced into the host cell).

The phrase "regulatory sequence" is intended to includes promoters, enhancers and other expression control elements (*e*.*g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e*.*g*., SECP proteins, mutant forms of SECP proteins, fusion proteins, etc.).

The recombinant expression vectors of the invention can be designed for expression of SECP proteins in prokaryotic or eukaryotic cells. For example, SECP proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T₇ promoter regulatory sequences and T₇ polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xₐ, thrombin, and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *Escherichia coli* expression vectors include pTrc (Amrann *et al*., (1988) *Gene* 69:301-315) and pET 11d (Studier, *et al*., GENE EXPRESSION TECHNOLOGY: METHODS IN ENZYMOLOGY 185, Academic Press, San Diego, Calif. (1990) 60-89).

One strategy to maximize recombinant protein expression in *Escherichia coli* is to express the protein in a host bacteria with an impaired capacity to proteolytically-cleave the recombinant protein, *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *Escherichia coli* (*see, e.g.,* Wada, *et al*., 1992. *Nucl*. *Acids Res.* 20: 2111-2118). Such alteration of nucleic acid sequences of the invention can be carried out by standard DNA synthesis techniques.

In another embodiment, the SECP expression vector is a yeast expression vector. Examples of vectors for expression in yeast *Saccharomyces cerivisae* include pYepSec1 (Baldari, *et al*., 1987. *EMBO J*. 6: 229-234), pMFa (Kurjan and Herskowitz, 1982. *Cell* 30: 933-943), pJRY88 (Schultz *et al*., 1987. *Gene* 54: 113-123), pYES2 (Invitrogen Corporation, San Diego, Calif.), and picZ (InVitrogen, Corp.; San Diego, Calif.).

Alternatively, SECP can be expressed in insect cells using baculovirus expression vectors. Baculovirus vectors available for expression of proteins in cultured insect cells *(e.g.,* SF9 cells) include the pAc series (Smith, *et al.,* 1983. *Mol. Cell. Biol*. 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaufman, *et al*., 1987. *EMBO J*. 6: 187-195). When used in mammalian cells, the expression vector's control functions are often provided by viral regulatory elements. For example, commonly used promoters are derived from polyoma, adenovirus 2, cytomegalovirus, and simian virus 40 (SV 40). For other suitable expression systems for both prokaryotic and eukaryotic cells *see, e.g.,* Chapters 16 and 17 of Sambrook, *et al*., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; *see*, Pinkert, *et al*., 1987. *Genes Dev*. 1: 268-277), lymphoid-specific promoters (*see*, Calame and Eaton, 1988. *Adv*. *Immunol*. *43:* 235-275), in particular promoters of T cell receptors (*see*, Winoto and Baltimore, 1989. *EMBO J*. 8: 729-733) and immunoglobulins (*see*, Banerji, *et al.,* 1983. *Cell* 33: 729-740; Queen and Baltimore, 1983. *Cell* 33: 741-748), neuron-specific promoters (*e*.*g*., the neurofilament promoter; *see,* Byrne and Ruddle, 1989. *Proc. Natl. Acad. Sci. USA* 86: 5473-5477), pancreas-specific promoters (*see*, Edlund, *et al.,* 1985. *Science* 230: 912-916), and mammary gland-specific promoters (*e*.*g*., milk whey promoter; U.S. Pat. No. 4,873,316 and European Application Publication No. 264,166). Developmentally-regulated promoters are also encompassed, *e*.*g*., the murine hox promoters (Kessel and Gruss, 1990. *Science* 249: 374-379) and the α-fetoprotein promoter (*see,* Campes and Tilghman, 1989. *Genes Dev.* 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to SECP mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al.,* "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, SECP protein can be expressed in bacterial cells such as *Escherichia coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding SECP or can be introduced on a separate vector. Cells stably-transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (*i*.*e*., express) SECP protein. Accordingly, the invention further provides methods for producing SECP protein using the host cells of the invention. In one embodiment, the method comprises culturing the host cell of invention (*i*.*e*., into which a recombinant expression vector encoding SECP protein has been introduced) in a suitable medium such that SECP protein is produced. In another embodiment, the method further comprises isolating SECP protein from the medium or the host cell.

### Transgenic Animals

The host cells of the invention can also be used to produce non-human transgenic animals. For example, in one embodiment, a host cell of the invention is a fertilized oocyte or an embryonic stem cell into which SECP protein-coding sequences have been introduced. These host cells can then be used to create non-human transgenic animals in which exogenous SECP sequences have been introduced into their genome or homologous recombinant animals in which endogenous SECP sequences have been altered. Such animals are useful for studying the function and/or activity of SECP protein and for identifying and/or evaluating modulators of SECP protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc.

A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous SECP gene has been altered by homologous recombination between the endogenous gene and an exogenous DNA molecule introduced into a cell of the animal, *e*.*g*., an embryonic cell of the animal, prior to development of the animal.

A transgenic animal of the invention can be created by introducing SECP-encoding nucleic acid into the male pronuclei of a fertilized oocyte (*e*.*g*., by micro-injection, retroviral infection) and allowing the oocyte to develop in a pseudopregnant female foster animal. The human SECP cDNA sequences of SEQ ID NO:1, 3, 5, 7, 9, 11, 13, 15, and/or 17, can be introduced as a transgene into the genome of a non-human animal. Alternatively, a non-human homologue of the human SECP gene, such as a mouse SECP gene, can be isolated based on hybridization to the human SECP cDNA (described further *supra*) and used as a transgene. Intronic sequences and polyadenylation signals can also be included in the transgene to increase the efficiency of expression of the transgene. A tissue-specific regulatory sequence(s) can be operably-linked to the SECP transgene to direct expression of SECP protein to particular cells. Methods for generating transgenic animals via embryo manipulation and micro-injection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the SECP transgene in its genome and/or expression of SECP mRNA in tissues or cells of the animals. A transgenic founder animal can then be used to breed additional animals carrying the transgene. Moreover, transgenic animals carrying a transgene-encoding SECP protein can further be bred to other transgenic animals carrying other transgenes.

To create a homologous recombinant animal, a vector is prepared which contains at least a portion of a SECP gene into which a deletion, addition or substitution has been introduced to thereby alter, *e*.*g*., functionally disrupt, the SECP gene. The SECP gene can be a human gene (*e*.*g*., the cDNA of SEQ ID NO:1, 3, 5, 7, 9, 11, 13, 15, and/or 17), but more preferably, is a non-human homologue of a human SECP gene. For example, a mouse homologue of human SECP gene of SEQ ID NO:1, 3, 5, 7, 9, 11, 13, 15, and/or 17, can be used to construct a homologous recombination vector suitable for altering an endogenous SECP gene in the mouse genome. In one embodiment, the vector is designed such that, upon homologous recombination, the endogenous SECP gene is functionally disrupted (*i*.*e*., no longer encodes a functional protein; also referred to as a "knock out" vector).

Alternatively, the vector can be designed such that, upon homologous recombination, the endogenous SECP gene is mutated or otherwise altered but still encodes functional protein (e.g., the upstream regulatory region can be altered to thereby alter the expression of the endogenous SECP protein). In the homologous recombination vector, the altered portion of the SECP gene is flanked at its 5'- and 3'-termini by additional nucleic acid of the SECP gene to allow for homologous recombination to occur between the exogenous SECP gene carried by the vector and an endogenous SECP gene in an embryonic stem cell. The additional flanking SECP nucleic acid is of sufficient length for successful homologous recombination with the endogenous gene. Typically, several kilobases (Kb) of flanking DNA (both at the 5'- and 3'-termini) are included in the vector. *See, e.g.,* Thomas, *et al.,* 1987. *Cell* 51: 503 for a description of homologous recombination vectors. The vector is ten introduced into an embryonic stem cell line (*e*.*g*., by electroporation) and cells in which the introduced SECP gene has homologously-recombined with the endogenous SECP gene are selected. *See, e.g.,* Li, *et al.,* 1992. *Cell* 69: 915.

The selected cells are then micro-injected into a blastocyst of an animal (*e*.*g*., a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr*. *Opin. Biotechnol.* 2: 823-829; PCT International Publication Nos.: WO 90/11354; WO 91/01140; WO 92/0968; and WO 93/04169.

In another embodiment, transgenic non-human animals can be produced that contain selected systems that allow for regulated expression of the transgene. One example of such a system is the cre/loxP recombinase system of bacteriophage P1. For a description of the cre/loxP recombinase system, *See, e.g.,* Lakso, *et al.,* 1992. *Proc. Natl*. *Acad*. *Sci*. *USA 89:* 6232-6236. Another example of a recombinase system is the FLP recombinase system of *Saccharomyces cerevisiae*. *See,* O'Gorman, *et al.,* 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e*.*g*., by mating two transgenic animals, one containing a transgene encoding a selected protein and the other containing a transgene encoding a recombinase.

Clones of the non-human transgenic animals described herein can also be produced according to the methods described in Wilmut, *et al*., 1997. *Nature* 385: 810-813. In brief, a cell (*e*.*g*., a somatic cell) from the transgenic animal can be isolated and induced to exit the growth cycle and enter G₀ phase. The quiescent cell can then be fused, *e*.*g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (*e*.*g*., the somatic cell) is isolated.

### Pharmaceutical Compositions

The SECP nucleic acid molecules, SECP proteins, and anti-SECP antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically-acceptable carrier. As used herein, "pharmaceutically-acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and other non-aqueous (*i*.*e*., lipophilic) vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e*.*g*., intravenous, intradermal, subcutaneous, oral (*e*.*g*., inhalation), transdermal (*i*.*e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e*.*g*., a SECP protein or anti-SECP antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e*.*g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e.g.,* U.S. Patent No. 5,328,470) or by stereotactic injection (*see, e*.*g*., Chen, *et al.,* 1994. *Proc. Natl*. *Acad*. *Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

The pharmaceutical compositions can be included in a container, pack, or dispenser together with instructions for administration.

### Screening and Detection Methods

The nucleic acid molecules, proteins, protein homologues, and antibodies described herein can be used in one or more of the following methods: (A) screening assays; (B) detection assays (*e*.*g*., chromosomal mapping, cell and tissue typing, forensic biology), (C) predictive medicine (*e*.*g*., diagnostic assays, prognostic assays, monitoring clinical trials, and pharmacogenomics); and (D) methods of treatment (*e*.*g*., therapeutic and prophylactic).

The isolated nucleic acid molecules of the present invention can be used to express SECP protein (*e*.*g*., via a recombinant expression vector in a host cell in gene therapy applications), to detect SECP mRNA (*e*.*g*., in a biological sample) or a genetic lesion in an SECP gene, and to modulate SECP activity, as described further below. In addition, the SECP proteins can be used to screen drugs or compounds that modulate the SECP protein activity or expression as well as to treat disorders characterized by insufficient or excessive production of SECP protein or production of SECP protein forms that have decreased or aberrant activity compared to SECP wild-type protein. In addition, the anti-SECP antibodies of the present invention can be used to detect and isolate SECP proteins and modulate SECP activity.

The invention further pertains to novel agents identified by the screening assays described herein and uses thereof for treatments as previously described.

### Screening Assays

The invention provides a method (also referred to herein as a "screening assay") for identifying modulators, i.e., candidate or test compounds or agents (e.g., peptides, peptidomimetics, small molecules or other drugs) that bind to SECP proteins or have a stimulatory or inhibitory effect on, *e*.*g*., SECP protein expression or SECP protein activity. The invention also includes compounds identified in the screening assays described herein.

In one embodiment, the invention provides assays for screening candidate or test compounds which bind to or modulate the activity of the membrane-bound form of a SECP protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e*.*g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, *et al.,* 1993. *Proc. Natl. Acad. Sci. U*.*S*.*A*. 90: 6909; Erb, *et al.,* 1994. *Proc*. *Natl. Acad*. *Sci. U*.*S*.*A*. 91: 11422; Zuckermann, *et al.,* 1994. *J*. *Med. Chem.* 37: 2678; Cho, *et al.,* 1993. *Science* 261: 1303; Carrell, *et al*., 1994. *Angew. Chem. Int. Ed*. *Engl*. 33: 2059; Carell, *et al.,* 1994. *Angew. Chem. Int. Ed. Engl.* 33: 2061; and Gallop, *et al.,* 1994. *J*. *Med*. *Chem.* 37: 1233.

Libraries of compounds may be presented in solution (*e*.*g*., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids (Cull, *et al*., 1992. *Proc. Natl*. *Acad*. *Sci. USA* 89: 1865-1869) or on phage (Scott and Smith, 1990. *Science* 249: 386-390; Devlin, 1990. *Science* 249: 404-406; Cwirla, *et al*., 1990. *Proc. Natl*. *Acad*. *Sci. U*.*S*.*A*. 87: 6378-6382; Felici, 1991. *J*. *Mol. Biol*. 222: 301-310; Ladner, U.S. Patent No. 5,233,409.).

In one embodiment, an assay is a cell-based assay in which a cell which expresses a membrane-bound form of SECP protein, or a biologically-active portion thereof, on the cell surface is contacted with a test compound and the ability of the test compound to bind to a SECP protein determined. The cell, for example, can of mammalian origin or a yeast cell. Determining the ability of the test compound to bind to the SECP protein can be accomplished, for example, by coupling the test compound with a radioisotope or enzymatic label such that binding of the test compound to the SECP protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of SECP protein, or a biologically-active portion thereof, on the cell surface with a known compound which binds SECP to form an assay mixture, contacting the assay mixture with a test compound, and determining the ability of the test compound to interact with a SECP protein, wherein determining the ability of the test compound to interact with a SECP protein comprises determining the ability of the test compound to preferentially bind to SECP protein or a biologically-active portion thereof as compared to the known compound.

In another embodiment, an assay is a cell-based assay comprising contacting a cell expressing a membrane-bound form of SECP protein, or a biologically-active portion thereof, on the cell surface with a test compound and determining the ability of the test compound to modulate (*e*.*g*., stimulate or inhibit) the activity of the SECP protein or biologically-active portion thereof. Determining the ability of the test compound to modulate the activity of SECP or a biologically-active portion thereof can be accomplished, for example, by determining the ability of the SECP protein to bind to or interact with a SECP target molecule. As used herein, a "target molecule" is a molecule with which a SECP protein binds or interacts in nature, for example, a molecule on the surface of a cell which expresses a SECP interacting protein, a molecule on the surface of a second cell, a molecule in the extracellular *milieu*, a molecule associated with the internal surface of a cell membrane or a cytoplasmic molecule. An SECP target molecule can be a non-SECP molecule or a SECP protein or polypeptide of the invention. In one embodiment, a SECP target molecule is a component of a signal transduction pathway that facilitates transduction of an extracellular signal (*e*.*g*. a signal generated by binding of a compound to a membrane-bound SECP molecule) through the cell membrane and into the cell. The target, for example, can be a second intercellular protein that has catalytic activity or a protein that facilitates the association of downstream signaling molecules with SECP.

Determining the ability of the SECP protein to bind to or interact with a SECP target molecule can be accomplished by one of the methods described above for determining direct binding. In one embodiment, determining the ability of the SECP protein to bind to or interact with a SECP target molecule can be accomplished by determining the activity of the target molecule. For example, the activity of the target molecule can be determined by detecting induction of a cellular second messenger of the target (*i*.*e*. intracellular Ca²⁺, diacylglycerol, IP₃, etc.), detecting catalytic/enzymatic activity of the target an appropriate substrate, detecting the induction of a reporter gene (comprising a SECP-responsive regulatory element operatively linked to a nucleic acid encoding a detectable marker, *e*.*g*., luciferase), or detecting a cellular response, for example, cell survival, cellular differentiation, or cell proliferation.

In yet another embodiment, an assay of the invention is a cell-free assay comprising contacting a SECP protein or biologically-active portion thereof with a test compound and determining the ability of the test compound to bind to the SECP protein or biologically-active portion thereof. Binding of the test compound to the SECP protein can be determined either directly or indirectly as described above. In one such embodiment, the assay comprises contacting the SECP protein or biologically-active portion thereof with a known compound which binds SECP to form an assay mixture, contacting the assay mixture with a test compound, and determining the ability of the test compound to interact with a SECP protein, wherein determining the ability of the test compound to interact with a SECP protein comprises determining the ability of the test compound to preferentially bind to SECP or biologically-active portion thereof as compared to the known compound.

In still another embodiment, an assay is a cell-free assay comprising contacting SECP protein or biologically-active portion thereof with a test compound and determining the ability of the test compound to modulate (*e*.*g*. stimulate or inhibit) the activity of the SECP protein or biologically-active portion thereof. Determining the ability of the test compound to modulate the activity of SECP can be accomplished, for example, by determining the ability of the SECP protein to bind to a SECP target molecule by one of the methods described above for determining direct binding. In an alternative embodiment, determining the ability of the test compound to modulate the activity of SECP protein can be accomplished by determining the ability of the SECP protein further modulate a SECP target molecule. For example, the catalytic/enzymatic activity of the target molecule on an appropriate substrate can be determined as described, *supra.*

In yet another embodiment, the cell-free assay comprises contacting the SECP protein or biologically-active portion thereof with a known compound which binds SECP protein to form an assay mixture, contacting the assay mixture with a test compound, and determining the ability of the test compound to interact with a SECP protein, wherein determining the ability of the test compound to interact with a SECP protein comprises determining the ability of the SECP protein to preferentially bind to or modulate the activity of a SECP target molecule.

The cell-free assays of the invention are amenable to use of both the soluble form or the membrane-bound form of SECP protein. In the case of cell-free assays comprising the membrane-bound form of SECP protein, it may be desirable to utilize a solubilizing agent such that the membrane-bound form of SECP protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either SECP protein or its target molecule to facilitate separation of complexed from uncomplexed forms of one or both of the proteins, as well as to accommodate automation of the assay. Binding of a test compound to SECP protein, or interaction of SECP protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-SECP fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or SECP protein, and the mixture is incubated under conditions conducive to complex formation (*e*.*g*., at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of SECP protein binding or activity determined using standard techniques.

Other techniques for immobilizing proteins on matrices can also be used in the screening assays of the invention. For example, either the SECP protein or its target molecule can be immobilized utilizing conjugation ofbiotin and streptavidin. Biotinylated SECP protein or target molecules can be prepared from biotin-NHS (N-hydroxy-succinimide) using techniques well-known within the art (*e*.*g*., biotinylation kit, Pierce Chemicals, Rockford, Ill.), and immobilized in the wells of streptavidin-coated 96 well plates (Pierce Chemical). Alternatively, antibodies reactive with SECP protein or target molecules, but which do not interfere with binding of the SECP protein to its target molecule, can be derivatized to the wells of the plate, and unbound target or SECP protein trapped in the wells by antibody conjugation. Methods for detecting such complexes, in addition to those described above for the GST-immobilized complexes, include immunodetection of complexes using antibodies reactive with the SECP protein or target molecule, as well as enzyme-linked assays that rely on detecting an enzymatic activity associated with the SECP protein or target molecule.

In another embodiment, modulators of SECP protein expression are identified in a method wherein a cell is contacted with a candidate compound and the expression of SECP mRNA or protein in the cell is determined. The level of expression of SECP mRNA or protein in the presence of the candidate compound is compared to the level of expression of SECP mRNA or protein in the absence of the candidate compound. The candidate compound can then be identified as a modulator of SECP mRNA or protein expression based upon this comparison. For example, when expression of SECP mRNA or protein is greater (*i*.*e*., statistically significantly greater) in the presence of the candidate compound than in its absence, the candidate compound is identified as a stimulator of SECP mRNA or protein expression. Alternatively, when expression of SECP mRNA or protein is less (statistically significantly less) in the presence of the candidate compound than in its absence, the candidate compound is identified as an inhibitor of SECP mRNA or protein expression. The level of SECP mRNA or protein expression in the cells can be determined by methods described herein for detecting SECP mRNA or protein.

In yet another aspect of the invention, the SECP proteins can be used as "bait proteins" in a two-hybrid assay or three hybrid assay (*see*, *e*.*g*., U.S. Patent No. 5,283,317; Zervos, *et al*., 1993. *Cell* 72: 223-232; Madura, *et al*., 1993. *J*. *Biol*. *Chem.* 268: 12046-12054; Bartel, *et al*., 1993. *Biotechniques* 14: 920-924; Iwabuchi, *et al.,* 1993. *Oncogene* 8: 1693-1696; and Brent WO 94/10300), to identify other proteins that bind to or interact with SECP ("SECP-binding proteins" or "SECP-bp") and modulate SECP activity. Such SECP-binding proteins are also likely to be involved in the propagation of signals by the SECP proteins as, for example, upstream or downstream elements of the SECP pathway.

The two-hybrid system is based on the modular nature of most transcription factors, which consist of separable DNA-binding and activation domains. Briefly, the assay utilizes two different DNA constructs. In one construct, the gene that codes for SECP is fused to a gene encoding the DNA binding domain of a known transcription factor (*e*.*g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a SECP-dependent complex, the DNA-binding and activation domains of the transcription factor are brought into close proximity. This proximity allows transcription of a reporter gene (*e*.*g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with SECP.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (*ii*) identify an individual from a minute biological sample (tissue typing); and (*iii*) aid in forensic identification of a biological sample. Some of these applications are described in the subsections below.

### Chromosome Mapping

Once the sequence (or a portion of the sequence) of a gene has been isolated, this sequence can be used to map the location of the gene on a chromosome. This process is called chromosome mapping. Accordingly, portions or fragments of the SECP sequences shown in SEQ ID NO:1, 3, 5, 7, 9, 11, 13, 15, and/or 17, or fragments or derivatives thereof, can be used to map the location of the SECP genes, respectively, on a chromosome. The mapping of the SECP sequences to chromosomes is an important first step in correlating these sequences with genes associated with disease.

Briefly, SECP genes can be mapped to chromosomes by preparing PCR primers (preferably 15-25 bp in length) from the SECP sequences. Computer analysis of the SECP, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the SECP sequences will yield an amplified fragment.

Somatic cell hybrids are prepared by fusing somatic cells from different mammals (*e*.*g*., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e*.*g*., D'Eustachio, *et al*., 1983. *Science* 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the SECP sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, see, Verma, *et al*., HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to non-coding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, *e*.*g*., in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e*.*g*., Egeland, *et al*., 1987. *Nature,* 325: 783-787.

Additionally, differences in the DNA sequences between individuals affected and unaffected with a disease associated with the SECP gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The SECP sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," as described in U.S. Patent No. 5,272,057).

Furthermore, the sequences of the invention can be used to provide an alternative technique that determines the actual base-by-base DNA sequence of selected portions of an individual's genome. Thus, the SECP sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The SECP sequences of the invention uniquely represent portions of the human genome. Allelic variation occurs to some degree in the coding regions of these sequences, and to a greater degree in the non-coding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the non-coding regions, fewer sequences are necessary to differentiate individuals. The non-coding sequences can comfortably provide positive individual identification with a panel of perhaps 10 to 1,000 primers that each yield a non-coding amplified sequence of 100 bases. If predicted coding sequences, such as those in SEQ ID NO:1, 3, 5, 7, 9, 11, 13, 15, and/or 17 are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining SECP protein and/or nucleic acid expression as well as SECP activity, in the context of a biological sample *(e.g.,* blood, serum, cells, tissue) to thereby determine whether an individual is afflicted with a disease or disorder, or is at risk of developing a disorder, associated with aberrant SECP expression or activity. The invention also provides for prognostic (or predictive) assays for determining whether an individual is at risk of developing a disorder associated with SECP protein, nucleic acid expression or activity. For example, mutations in a SECP gene can be assayed in a biological sample. Such assays can be used for prognostic or predictive purpose to thereby prophylactically treat an individual prior to the onset of a disorder characterized by or associated with SECP protein, nucleic acid expression or activity.

Another aspect of the invention provides methods for determining SECP protein, nucleic acid expression or SECP activity in an individual to thereby select appropriate therapeutic or prophylactic agents for that individual (referred to herein as "pharmacogenomics"). Pharmacogenomics allows for the selection of agents (*e*.*g*., drugs) for therapeutic or prophylactic treatment of an individual based on the genotype of the individual (*e*.*g*., the genotype of the individual examined to determine the ability of the individual to respond to a particular agent.)

Yet another aspect of the invention pertains to monitoring the influence of agents (*e*.*g*., drugs, compounds) on the expression or activity of SECP in clinical trials.

### Use of Partial SECP Sequences in Forensic Biology

DNA-based identification techniques can also be used in forensic biology. Forensic biology is a scientific field employing genetic typing of biological evidence found at a crime scene as a means for positively identifying, *e*.*g*., a perpetrator of a crime. To make such an identification, PCR technology can be used to amplify DNA sequences taken from very small biological samples such as tissues (*e*.*g*., hair or skin, or body fluids, *e.g.,* blood, saliva, or semen found at a crime scene). The amplified sequence can then be compared to a standard, thereby allowing identification of the origin of the biological sample.

The sequences of the invention can be used to provide polynucleotide reagents, *e*.*g*., PCR primers, targeted to specific loci in the human genome, that can enhance the reliability of DNA-based forensic identifications by, for example, providing another "identification marker" (*i*.*e*. another DNA sequence that is unique to a particular individual). As mentioned above, actual base sequence information can be used for identification as an accurate alternative to patterns formed by restriction enzyme generated fragments. Sequences targeted to non-coding regions of SEQ ID NO:1, 3, 5, 7, 9, 11, 13, 15, and/or 17 are particularly appropriate for this use as greater numbers of polymorphisms occur in the non-coding regions, making it easier to differentiate individuals using this technique. Examples of polynucleotide reagents include the SECP sequences or portions thereof, *e*.*g*., fragments derived from the non-coding regions of one or more of SEQ ID NO:1, 3, 5, 7, 9, 11, 13, 15, and/or 17, having a length of at least 20 bases, preferably at least 30 bases.

The SECP sequences described herein can further be used to provide polynucleotide reagents, *e*.*g*., labeled or label-able probes that can be used, for example, in an *in situ* hybridization technique, to identify a specific tissue (*e*.*g*., brain tissue, etc). This can be very useful in cases where a forensic pathologist is presented with a tissue of unknown origin. Panels of such SECP probes can be used to identify tissue by species and/or by organ type.

In a similar fashion, these reagents, *e*.*g*., SECP primers or probes can be used to screen tissue culture for contamination (*i*.*e*., screen for the presence of a mixture of different types of cells in a culture).

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining SECP protein and/or nucleic acid expression as well as SECP activity, in the context of a biological sample (*e*.*g*., blood, serum, cells, tissue) to thereby determine whether an individual is afflicted with a disease or disorder, or is at risk of developing a disorder, associated with aberrant SECP expression or activity. The invention also provides for prognostic (or predictive) assays for determining whether an individual is at risk of developing a disorder associated with SECP protein, nucleic acid expression or activity. For example, mutations in a SECP gene can be assayed in a biological sample. Such assays can be used for prognostic or predictive purpose to thereby prophylactically treat an individual prior to the onset of a disorder characterized by or associated with SECP protein, nucleic acid expression, or biological activity.

Another aspect of the invention provides methods for determining SECP protein, nucleic acid expression or activity in an individual to thereby select appropriate therapeutic or prophylactic agents for that individual (referred to herein as "pharmacogenomics"). Pharmacogenomics allows for the selection of agents (*e*.*g*., drugs) for therapeutic or prophylactic treatment of an individual based on the genotype of the individual (*e*.*g*., the genotype of the individual examined to determine the ability of the individual to respond to a particular agent.)

Yet another aspect of the invention pertains to monitoring the influence of agents (*e*.*g*., drugs, compounds) on the expression or activity of SECP in clinical trials.

These and various other agents are described in further detail in the following sections.

### Diagnostic Assays

An exemplary method for detecting the presence or absence of SECP in a biological sample involves obtaining a biological sample from a test subject and contacting the biological sample with a compound or an agent capable of detecting SECP protein or nucleic acid (*e*.*g*., mRNA, genomic DNA) that encodes SECP protein such that the presence of SECP is detected in the biological sample. An agent for detecting SECP mRNA or genomic DNA is a labeled nucleic acid probe capable of hybridizing to SECP mRNA or genomic DNA. The nucleic acid probe can be, for example, a full-length SECP nucleic acid, such as the nucleic acid of SEQ ID NO:1, 3, 5, 7, 9, 11, 13, 15, and/or 17, or a portion thereof, such as an oligonucleotide of at least 15, 30, 50, 100, 250 or 500 nucleotides in length and sufficient to specifically hybridize under stringent conditions to SECP mRNA or genomic DNA. Other suitable probes for use in the diagnostic assays of the invention are described herein.

An agent for detecting SECP protein is an antibody capable of binding to SECP protein, preferably an antibody with a detectable label. Antibodies can be polyclonal, or more preferably, monoclonal. An intact antibody, or a fragment thereof (*e*.*g*., F_{ab} or F_{(ab)2}) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect SECP mRNA, protein, or genomic DNA in a biological sample *in vitro* as well as *in vivo.* For example, *in vitro* techniques for detection of SECP mRNA include Northern hybridizations and *in situ* hybridizations. *In vitro* techniques for detection of SECP protein include enzyme linked immunosorbent assays (ELISAs), Western blots, immunoprecipitations, and immunofluorescence. *In vitro* techniques for detection of SECP genomic DNA include Southern hybridizations. Furthermore, *in vivo* techniques for detection of SECP protein include introducing into a subject a labeled anti-SECP antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting SECP protein, mRNA, or genomic DNA, such that the presence of SECP protein, mRNA or genomic DNA is detected in the biological sample, and comparing the presence of SECP protein, mRNA or genomic DNA in the control sample with the presence of SECP protein, mRNA or genomic DNA in the test sample.

The invention also encompasses kits for detecting the presence of SECP in a biological sample. For example, the kit can comprise: a labeled compound or agent capable of detecting SECP protein or mRNA in a biological sample; means for determining the amount of SECP in the sample; and means for comparing the amount of SECP in the sample with a standard. The compound or agent can be packaged in a suitable container. The kit can further comprise instructions for using the kit to detect SECP protein or nucleic acid.

### Prognostic Assays

The diagnostic methods described herein can furthermore be utilized to identify subjects having or at risk of developing a disease or disorder associated with aberrant SECP expression or activity. For example, the assays described herein, such as the preceding diagnostic assays or the following assays, can be utilized to identify a subject having or at risk of developing a disorder associated with SECP protein, nucleic acid expression or activity. Alternatively, the prognostic assays can be utilized to identify a subject having or at risk for developing a disease or disorder. Thus, the invention provides a method for identifying a disease or disorder associated with aberrant SECP expression or activity in which a test sample is obtained from a subject and SECP protein or nucleic acid (*e*.*g*., mRNA, genomic DNA) is detected, wherein the presence of SECP protein or nucleic acid is diagnostic for a subject having or at risk of developing a disease or disorder associated with aberrant SECP expression or activity. As used herein, a "test sample" refers to a biological sample obtained from a subject of interest. For example, a test sample can be a biological fluid (*e*.*g*., serum), cell sample, or tissue.

Furthermore, the prognostic assays described herein can be used to determine whether a subject can be administered an agent (*e.g*., an agonist, antagonist, peptidomimetic, protein, peptide, nucleic acid, small molecule, or other drug candidate) to treat a disease or disorder associated with aberrant SECP expression or activity. For example, such methods can be used to determine whether a subject can be effectively treated with an agent for a disorder. Thus, the invention provides methods for determining whether a subject can be effectively treated with an agent for a disorder associated with aberrant SECP expression or activity in which a test sample is obtained and SECP protein or nucleic acid is detected (*e*.*g*., wherein the presence of SECP protein or nucleic acid is diagnostic for a subject that can be administered the agent to treat a disorder associated with aberrant SECP expression or activity).

The methods of the invention can also be used to detect genetic lesions in a SECP gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a SECP-protein, or the mis-expression of the SECP gene. For example, such genetic lesions can be detected by ascertaining the existence of at least one of: (*i*) a deletion of one or more nucleotides from a SECP gene; (*ii*) an addition of one or more nucleotides to a SECP gene;
(*iii*) a substitution of one or more nucleotides of a SECP gene, (*iv*) a chromosomal rearrangement of a SECP gene; (*v*) an alteration in the level of a messenger RNA transcript of a SECP gene,
(*vi*) aberrant modification of a SECP gene, such as of the methylation pattern of the genomic DNA, (*vii*) the presence of a non-wild-type splicing pattern of a messenger RNA transcript of a SECP gene, *(viii)* a non-wild-type level of a SECP protein, (*ix*) allelic loss of a SECP gene, and (x) inappropriate post-translational modification of a SECP protein. As described herein, there are a large number of assay techniques known in the art which can be used for detecting lesions in a SECP gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) (*see, e.g.,* U.S. Patent Nos. 4,683,195 and 4,683,202), such as anchor PCR or RACE PCR, or, alternatively, in a ligation chain reaction (LCR) (*see, e.g.,* Landegran, *et al*., 1988. *Science* 241: 1077-1080; and Nakazawa, *et al.,* 1994. *Proc. Natl*. *Acad*. *Sci. USA* 91: 360-364), the latter of which can be particularly useful for detecting point mutations in the SECP-gene (*see,* Abravaya, *et al*., 1995. *Nucl*. *Acids Res.* 23: 675-682). This method can include the steps of collecting a sample of cells from a patient, isolating nucleic acid (*e*.*g*., genomic, mRNA or both) from the cells of the sample, contacting the nucleic acid sample with one or more primers that specifically hybridize to a SECP gene under conditions such that hybridization and amplification of the SECP gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see,* Guatelli, *et al*., 1990. *Proc. Natl*. *Acad. Sci. USA* 87: 1874-1878), transcriptional amplification system (*see,* Kwoh, *et al.,* 1989. *Proc. Natl*. *Acad*. *Sci. USA* 86: 1173-1177); Qβ Replicase (*see,* Lizardi, *et al*, 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a SECP gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in SECP can be identified by hybridizing a sample and control nucleic acids, *e*.*g*., DNA or RNA, to high-density arrays containing hundreds or thousands of oligonucleotides probes. *See, e*.*g*., Cronin, *et al.,* 1996. *Human Mutation* 7: 244-255; Kozal, *et al., 1996. Nat. Med.* 2: 753-759. For example, genetic mutations in SECP can be identified in two dimensional arrays containing light-generated DNA probes as described in Cronin, *et al*., *supra.* Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the SECP gene and detect mutations by comparing the sequence of the sample SECP with the corresponding wild-type (control) sequence. Examples of sequencing reactions include those based on techniques developed by Maxim and Gilbert, 1977. *Proc. Natl*. *Acad. Sci. USA* 74: 560 or Sanger, 1977. *Proc. Natl*. *Acad*. *Sci. USA* 74: 5463. It is also contemplated that any of a variety of automated sequencing procedures can be utilized when performing the diagnostic assays *(see, e*.*g*., Naeve, *et al.,* 1995. *Biotechniques* 19: 448), including sequencing by mass spectrometry (see, *e*.*g*., PCT International Publication No. WO 94/16101; Cohen, *et al*., 1996. *Adv*. *Chromatography* 36: 127-162; and Griffin, *et al.,* 1993. *Appl*. *Biochem. Biotechnol.* 38: 147-159).

Other methods for detecting mutations in the SECP gene include methods in which protection from cleavage agents is used to detect mismatched bases in RNA/RNA or RNA/DNA heteroduplexes. *See, e*.*g*., Myers, *et al.,* 1985. *Science* 230: 1242. In general, the art technique of "mismatch cleavage" starts by providing heteroduplexes of formed by hybridizing (labeled) RNA or DNA containing the wild-type SECP sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e*.*g*., Cotton, *et al.,* 1988. *Proc. Natl*. *Acad. Sci. USA* 85: 4397; Saleeba, *et al*., 1992. *Methods Enzymol*. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in SECP cDNAs obtained from samples of cells. For example, the mutY enzyme of *E*. *coli* cleaves A at G/A mismatches and the thymidine DNA glycosylase from HeLa cells cleaves T at G/T mismatches. *See, e*.*g*., Hsu, *et al.,* 1994. *Carcinogenesis* 15: 1657-1662. According to an exemplary embodiment, a probe based on a SECP sequence, *e*.*g*., a wild-type SECP sequence, is hybridized to a cDNA or other DNA product from a test cell(s). The duplex is treated with a DNA mismatch repair enzyme, and the cleavage products, if any, can be detected from electrophoresis protocols or the like. *See, e*.*g*., U.S. Patent No. 5,459,039.

In other embodiments, alterations in electrophoretic mobility will be used to identify mutations in SECP genes. For example, single strand conformation polymorphism (SSCP) may be used to detect differences in electrophoretic mobility between mutant and wild type nucleic acids. *See, e*.*g*., Orita, *et al.,* 1989. *Proc. Natl. Acad*. *Sci. USA:* 86: 2766; Cotton, 1993. *Mutat. Res.* 285: 125-144; Hayashi, 1992. *Genet. Anal. Tech. Appl.* 9: 73-79. Single-stranded DNA fragments of sample and control SECP nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, *et al.,* 1991. *Trends Genet.* 7: 5.

In yet another embodiment, the movement of mutant or wild-type fragments in polyacrylamide gels containing a gradient of denaturant is assayed using denaturing gradient gel electrophoresis (DGGE). *See, e.g.,* Myers, *et al.,* 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.*, Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al.,* 1986. *Nature* 324: 163; Saiki, *et al*., 1989. *Proc. Natl*. *Acad*. *Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e*.*g*., Gibbs, *et al*., 1989. *Nucl. Acids Res.* 17: 2437-2448) or at the extreme 3'-terminus of one primer where, under appropriate conditions, mismatch can prevent, or reduce polymerase extension *(see, e.g.,* Prossner, 1993. *Tibtech*. 11: 238). In addition it may be desirable to introduce a novel restriction site in the region of the mutation to create cleavage-based detection. *See, e*.*g*., *Gasparini, et al*., 1992. *Mol*. *Cell Probes* 6: 1. It is anticipated that in certain embodiments amplification may also be performed using *Taq* ligase for amplification. *See, e.g.,* Barany, *1991. Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e*.*g*., in clinical settings to diagnose patients exhibiting symptoms or family history of a disease or illness involving a SECP gene.

Furthermore, any cell type or tissue, preferably peripheral blood leukocytes, in which SECP is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on SECP activity (*e*.*g*., SECP gene expression), as identified by a screening assay described herein can be administered to individuals to treat (prophylactically or therapeutically) disorders (*e*.*g*., cancer or immune disorders associated with aberrant SECP activity. In conjunction with such treatment, the pharmacogenomics (*i*.*e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e.g*., drugs) for prophylactic or therapeutic treatments based on a consideration of the individual's genotype. Such pharmacogenomics can further be used to determine appropriate dosages and therapeutic regimens. Accordingly, the activity of SECP protein, expression of SECP nucleic acid, or mutation content of SECP genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e.g.,* Eichelbaum, 1996. *Clin. Exp. Pharmacol*. *Physiol*. 23: 983-985; Linder, 1997. *Clin. Chem*., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e.g.,* N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of SECP protein, expression of SECP nucleic acid, or mutation content of SECP genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a SECP modulator, such as a modulator identified by one of the exemplary screening assays described herein.

### Monitoring of Effects During Clinical Trials

Monitoring the influence of agents (*e*.*g*., drugs, compounds) on the expression or activity of SECP (*e*.*g*., the ability to modulate aberrant cell proliferation and/or differentiation) can be applied not only in basic drug screening, but also in clinical trials. For example, the effectiveness of an agent determined by a screening assay as described herein to increase SECP gene expression, protein levels, or upregulate SECP activity, can be monitored in clinical trails of subjects exhibiting decreased SECP gene expression, protein levels, or down-regulated SECP activity. Alternatively, the effectiveness of an agent determined by a screening assay to decrease SECP gene expression, protein levels, or down-regulate SECP activity, can be monitored in clinical trails of subjects exhibiting increased SECP gene expression, protein levels, or up-regulated SECP activity. In such clinical trials, the expression or activity of SECP and, preferably, other genes that have been implicated in, for example, a cellular proliferation or immune disorder can be used as a "read out" or markers of the immune responsiveness of a particular cell.

By way of example, and not of limitation, genes, including SECP, that are modulated in cells by treatment with an agent (*e*.*g*., compound, drug or small molecule) that modulates SECP activity (*e*.*g*., identified in a screening assay as described herein) can be identified. Thus, to study the effect of agents on cellular proliferation disorders, for example, in a clinical trial, cells can be isolated and RNA prepared and analyzed for the levels of expression of SECP and other genes implicated in the disorder. The levels of gene expression (*i*.*e*., a gene expression pattern) can be quantified by Northern blot analysis or RT-PCR, as described herein, or alternatively by measuring the amount of protein produced, by one of the methods as described herein, or by measuring the levels of activity of SECP or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e.g.*, an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of a SECP protein, mRNA, or genomic DNA in the pre-administration sample; (*iii*) obtaining one or more post-administration samples from the subject; (*iv*) detecting the level of expression or activity of the SECP protein, mRNA, or genomic DNA in the post-administration samples; (*v*) comparing the level of expression or activity of the SECP protein, mRNA, or genomic DNA in the pre-administration sample with the SECP protein, mRNA, or genomic DNA in the post administration sample or samples; and (*vi*) altering the administration of the agent to the subject accordingly. For example, increased administration of the agent may be desirable to increase the expression or activity of SECP to higher levels than detected, *i*.*e*., to increase the effectiveness of the agent. Alternatively, decreased administration of the agent may be desirable to decrease expression or activity of SECP to lower levels than detected, *i*.*e*., to decrease the effectiveness of the agent.

### Methods of Treatment

The invention provides for both prophylactic and therapeutic methods of treating a subject at risk of (or susceptible to) a disorder or having a disorder associated with aberrant SECP expression or activity. These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i*.*e*., reduce or inhibit) activity. Therapeutics that antagonize activity may be administered in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i*.*e*., due to a heterologous insertion within the coding sequences of coding sequences to an aforementioned peptide) that are utilized to "knockout" endoggenous function of an aforementioned peptide by homologous recombination (*see, e.g.,* Capecchi, 1989. *Science* 244: 1288-1292); or (*v*) modulators (*i*.*e*., inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i*.*e*., are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e*.*g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e*.*g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e*.*g*., Northern assays, dot blots, *in situ* hybridization, and the like).

### Prophylactic Methods

In one aspect, the invention provides a method for preventing, in a subject, a disease or condition associated with an aberrant SECP expression or activity, by administering to the subject an agent that modulates SECP expression or at least one SECP activity. Subjects at risk for a disease that is caused or contributed to by aberrant SECP expression or activity can be identified by, for example, any or a combination of diagnostic or prognostic assays as described herein. Administration of a prophylactic agent can occur prior to the manifestation of symptoms characteristic of the SECP aberrancy, such that a disease or disorder is prevented or, alternatively, delayed in its progression. Depending upon the type of SECP aberrancy, for example, a SECP agonist or SECP antagonist agent can be used for treating the subject. The appropriate agent can be determined based on screening assays described herein.

### Therapeutic Methods

Another aspect of the invention pertains to methods of modulating SECP expression or activity for therapeutic purposes. The modulatory method of the invention involves contacting a cell with an agent that modulates one or more of the activities of SECP protein activity associated with the cell. An agent that modulates SECP protein activity can be an agent as described herein, such as a nucleic acid or a protein, a naturally-occurring cognate ligand of a SECP protein, a peptide, a SECP peptidomimetic, or other small molecule. In one embodiment, the agent stimulates one or more SECP protein activity. Examples of such stimulatory agents include active SECP protein and a nucleic acid molecule encoding SECP that has been introduced into the cell. In another embodiment, the agent inhibits one or more SECP protein activity. Examples of such inhibitory agents include antisense SECP nucleic acid molecules and anti-SECP antibodies. These modulatory methods can be performed in *vitro (e.g.,* by culturing the cell with the agent) or, alternatively, *in vivo (e.g.,* by administering the agent to a subject). As such, the invention provides methods of treating an individual afflicted with a disease or disorder characterized by aberrant expression or activity of a SECP protein or nucleic acid molecule. In one embodiment, the method involves administering an agent (e.g., an agent identified by a screening assay described herein), or combination of agents that modulates (*e*.*g*., up-regulates or down-regulates) SECP expression or activity. In another embodiment, the method involves administering a SECP protein or nucleic acid molecule as therapy to compensate for reduced or aberrant SECP expression or activity.

Stimulation of SECP activity is desirable in situations in which SECP is abnormally down-regulated and/or in which increased SECP activity is likely to have a beneficial effect. One example of such a situation is where a subject has a disorder characterized by aberrant cell proliferation and/or differentiation (*e*.*g*., cancer or immune associated disorders). Another example of such a situation is where the subject has a gestational disease (*e*.*g*., pre-clampsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The SECP nucleic acids and proteins of the invention may be useful in a variety of potential prophylactic and therapeutic applications. By way of a non-limiting example, a cDNA encoding the SECP protein of the invention may be useful in gene therapy, and the protein may be useful when administered to a subject in need thereof.

Both the novel nucleic acids encoding the SECP proteins, and the SECP proteins of the invention, or fragments thereof, may also be useful in diagnostic applications, wherein the presence or amount of the nucleic acid or the protein are to be assessed. These materials are further useful in the generation of antibodies which immunospecifically-bind to the novel substances of the invention for use in therapeutic or diagnostic methods.

The invention will be further illustrated in the following non-limiting examples.

### Example 1: Radiation Hybrid Mapping Provides the Chromosomal Location of SECP 2 (Clone 11618130.0.27)

Radiation hybrid mapping using human chromosome markers was carried out to determine the chromosomal location of a SECP2 nuclei acid of the invention. The procedure used to obtain these results is described generally in Steen, *et al*., 1999. A High-Density Integrated Genetic Linkage and Radiation Hybrid Map of the Laboratory Rat, *Genome Res.* 9: AP1-AP8 (Published Online on May 21, 1999). A panel of 93 cell clones containing randomized radiation-induced human chromosomal fragments was then screened in 96 well plates using PCR primers designed to identify the sought clones in a unique fashion. Clone 11618130.0.27, a SECP2 nucleic acid was located on chromosome 16 at a map distance of 26.0 cR from marker WI-3768 and -70.5 cR from marker TIGR-A002K05.

### Example 2: Molecular Cloning of Clone 11618130

Oligonucleotide PCR primers were designed to amplify a DNA segment coding for the full length open reading frame of clone 11618130. The forward primer included a Bgl II restriction site and the consensus Kozak sequence CCACC. The reverse primer contained an in-frame XhoI restriction site. Both primers contained a CTCGTC 5'-terminus clamp. The nucleotide sequences of the primers were:

11618130 Forward Primer:
CTCGTCAGATCTCCACCATGAGTGATGAGGACAGCTGTGTAG (SEQ ID NO:19)

11618130 Reverse Primer:
CTCGTCCTCGAGGCAGCTGGTTGGTTGGCTTATGTTG (SEQ ID NO:20)

The PCR reactions included: 5 ng human fetal brain cDNA template; 1 µM of each of the 11618130 Forward and 11618130 Reverse primers; 5 µM dNTP (Clontech Laboratories; Palo Alto, CA) and 1 µl of 50x Advantage-HF 2 polymerase (Clontech Laboratories; Palo Alto, CA) in 50 µl total reaction volume. The following PCR conditions were used:
a) 96°C 3 minutes
b) 96°C- 30 seconds denaturation
c) 70°C 30 seconds, primer annealing. This temperature was gradually decreased by 1°C/cycle
d) 72°C 1 minute extension.
Repeat steps b-d a total of 10-times
e) 96°C 30 seconds denaturation
f) 60°C 30 seconds annealing
g) 72°C 1 minute extension
Repeat steps e-g a total of 25-times
h) 72°C 5 minutes final extension

A single, amplified product of approximately 800 bp was detected by agarose gel electrophoresis. The PCR amplification product was then isolated by the QIAEX II® Gel Extraction System (QIAGEN, Inc; Valencia, CA) in a final volume of 20 µl.

A total of 10 µl of the isolated fragment was digested with Bgl II and XhoI restriction enzymes, and ligated into the BamHI- and XhoI-digested mammalian expression vector pCDNA3.1 V5His (Invitrogen; Carlsbad, CA.). The construct was sequenced, and the cloned insert was verified as a sequence identical to the ORF coding for the full length 11618130. The construct was designated pcDNA3.1-11618130-S178-2.

### Example 3: Expression of 11618130 In Human Embryonic Kidney 293 Cells

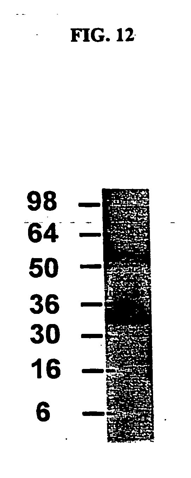

The vector pcDNA3.1-11618130-S178-2 described in Example 2 was subsequently transfected into human embryonic kidney 293 cells (ATCC No. CRL-1573; Manassas, VA) using the LipofectaminePlus Reagent following the manufacturer's instructions (Gibco/BRL/Life Technologies; Rockville, MD) The cell pellet and supernatant were harvested 72 hours after transfection, and examined for 11618130 expression by use of SDS-PAGE under reducing conditions and Western blotting with an anti-V5 antibody. FIG. 12 shows that 11618130 was expressed as a protein having an apparent molecular weight (Mr) of approximately 34 kilo Daltons (kDa) which was intracellularly expressed in the 293 cells. These experimental results were consistent with the predicted molecular weight of 28043 Daltons for the protein of clone 11618130.0.27 and with the predicted localization of the protein intracellularly in the microbody (peroxisome). A second band of approximately 54 kDa was also found, which may represent a non-reducible dimer of this protein.

### Example 4: Preparation of Mammalian Expression Vector pSecV5His

The oligonucleotide primers, pSec-V5-His Forward and pSec-V5-His Reverse, were generated to amplify a fragment from the pcDNA3.1-V5His (Invitrogen; Carlsbad, CA) expression vector that includes V5 and His6. The nucleotide sequences of these primers were:

pSec-V5-His Forward Primer:
CTCGTCCTCGAGGGTAAGCCTATCCCTAAC (SEQ ID NO:21)

pSec-V5-His Reverse Primer:
CTCGTCGGGCCCCTGATCAGCGGGTTTAAAC (SEQ ID NO:22)

The PCR product was digested with XhoI and ApaI, and ligated into the XhoI/ApaI-digested pSecTag2 B vector harboring an Ig kappa leader sequence (Invitrogen; Carlsbad, CA). The correct structure of the resulting vector (designated pSecV5His), including an in-frame
Ig-kappa leader and V5-His6, was verified by DNA sequence analysis. The pSecV5His vector included an in-frame Ig kappa leader, a site for insertion of a clone of interest, V5 and His6, which allows heterologous protein expression and secretion by fusing any protein to the Ig kappa chain signal peptide. Detection and purification of the expressed protein was aided by the presence of the V5 epitope tag and 6x His tag at the carboxyl-terminus (Invitrogen; Carlsbad, CA).

### Example 5: Molecular Cloning of 16406477

Oligonucleotide PCR primers were designed to amplify a DNA segment encoding for the mature form of clone 16406477 from amino acid residues 38 to 385, recognition of the signal sequence predicted for this polypeptide. The forward primer contained an in-frame BamHI restriction site and the reverse primer contained an in-frame XhoI restriction site.
Both primers contained the CTCGTC 5' clamp. The sequences of the primers were as follows:

16406477 Forward Primer:
CTCGTCGGATCCTGGGGCGCAGGGGAAGCCCCGGG (SEQ ID NO:23)

16406477 Reverse Primer:
CTCGTCCTCGAGGAGGGCAGCAAGGAGGCTGAGGGGCAG (SEQ ID NO:24)

The PCR reactions contained: 5 ng human fetal brain cDNA template; 1 µM of each of the 16406477 Forward and 16406477 Reverse Primers; 5 µM dNTP (Clontech Laboratories; Palo Alto, CA) and 1 µl of 50x Advantage-HF 2 polymerase (Clontech Laboratories; Palo Alto, CA) in a 50 µl total reaction volume. PCR was then conducted using reaction conditions identical to those previously described in Example 2.

A single, amplified product of approximately 1 Kbp was detected by agarose gel electrophoresis. The product was then isolated by QIAEX II® Gel Extraction System (QUIAGEN, Inc; Valencia, CA) in a total reaction volume of 20 µl.

A total of 10 µl of the isolated fragment was digested with BamHI and XhoI restriction enzymes, and ligated into the pSecV5-His mammalian expression vector *(see,* Example 4) which had been previously-digested with BamHI and XhoI. The construct was sequenced, and the cloned insert was verified as possessing a sequence identical to that of the ORF coding for the mature fragment of clone 16406477. The construct was subsequently designated pSecV5His-16406477-S196-A.

### Example 6: Expression of 16406477 in Human Embryonic Kidney 293 Cells

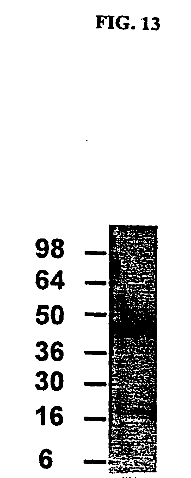

The pSecV5His-16406477-S196-A construct (see, Example 5) was subsequently transfected into 293 cells (ATCC No. CRL-1573; Manassas, VA) using the LipofectaminePlus Reagent following the manufacturer's instructions (Gibco/BRL/Life Technologies). The cell pellet and supernatant were harvested 72 hours after transfection, and examined for 16406477 expression by use of SDS-PAGE under reducing conditions and Western blotting with an anti-V5 antibody. FIG. 13 demonstrates that 16406477 is expressed as a protein having an apparent molecular weight (Mr) of approximately 45 kDa which is retained intracellularly in the 293 cells. The Mr value which was found upon expression of the clone is consistent with the predicted molecular weight of 43087 Daltons.

### Example 7: Quantitative Tissue Expression Analysis of Clones of the Invention

The Quantitative Expression Analysis of several clones of the invention was preformed in 41 normal and 55 tumor samples (*see,* FIG. 14) by real-time quantitative PCR (TAQMAN®) by use of a Perkin-Elmer Biosystems ABI PRISM® 7700 Sequence Detection System. The following abbreviations are used in FIG. 14:
ca. = carcinoma,
* = established from metastasis,
met = metastasis,
s cell var= small cell variant,
non-s = non-sm =non-small,
squam = squamous,
pl. eff = pl effusion = pleural effusion,
glio = glioma,
astro = astrocytoma, and
neuro = neuroblastoma.

Initially, 96 RNA samples were normalized to β-actin and GAPDH. RNA (∼50 ng total or ∼1 ng poly(A)+) was converted to cDNA using the TAQMAN® Reverse Transcription Reagents Kit (PE Biosystems; Foster City, CA; Catalog No. N808-0234) and random hexamers according to the manufacturer's protocol. Reactions were performed in a 20 µl total volume, and incubated for 30 minutes at 48°C. cDNA (5 µl) was then transferred to a separate plate for the TAQMAN® reaction using β-actin and GAPDH TAQMAN® Assay Reagents (PE Biosystems; Catalog Nos. 4310881E and 4310884E, respectively) and TAQMAN® Universal PCR Master Mix (PE Biosystems; Catalog No. 4304447) according to the manufacturer's protocol. Reactions were performed in a 25 µl total volume using the following parameters:
2 minutes at 50°C; 10 minutes at 95°C; 15 seconds at 95°C/1 min. at 60°C (40 cycles total).

Results were recorded as CT values (*i*.*e*., cycle at which a given sample crosses a threshold level of fluorescence) using a log scale, with the difference in RNA concentration between a given sample and the sample with the lowest CT value being represented as 2^{δCT}. The percent relative expression is then obtained by taking the reciprocal of this RNA difference and multiplying by 100. The average CT values obtained for β-actin and GAPDH were used to normalize RNA samples. The RNA sample generating the highest CT value required no further diluting, while all other samples were diluted relative to this sample according to their β-actin /GAPDH average CT values.

Normalized RNA (5 µl) was converted to cDNA and analyzed via TAQMAN® using One Step RT-PCR Master Mix Reagents (PE Biosystems; Catalog No. 4309169) and gene-specific primers according to the manufacturer's instructions. Probes and primers were designed for each assay according to Perkin Elmer Biosystem's Primer Express Software package (Version I for Apple Computer's Macintosh Power PC) using the sequence of the respective clones as input. Default settings were used for reaction conditions and the following parameters were set before selecting primers: primer concentration = 250 nM; primer melting temperature (Tₘ) range = 58°-60° C; primer optimal Tm = 59° C; maximum primer difference = 2° C, probe does not posses a 5'-terminus G; probe Tₘ must be 10° C greater than primer Tₘ; and amplicon size 75 bp to 100 bp in length. The probes and primers were synthesized by Synthegen (Houston, TX). Probes were double-purified by HPLC to remove uncoupled dye and then evaluated by mass spectroscopy to verify coupling of reporter and quencher dyes to the 5'- and 3'-termini of the probe, respectively. Their final concentrations used were - Forward and Reverse Primers = 900 nM each; and probe = 200nM.

Subsequent PCR conditions were as follows. Normalized RNA from each tissue and each cell line was spotted in each well of a 96 well PCR plate (Perkin Elmer Biosystems). PCR reaction mixes, including two probes (*i*.*e*., SECP-specific and another gene-specific probe multiplexed with the SEPC-specific probe) were set up using 1x TaqMan™ PCR Master Mix for the PE Biosystems 7700, with 5 mM MgCl₂; dNTPs (dA, G, C, U at 1:1:1:2 ratios); 0.25 U/ml AmpliTaq Gold™ (PE Biosystems); 0.4 U/µl RNase inhibitor; and 0.25 U/µl Reverse Transcriptase. Reverse transcription was then performed at 48°C for 30 minutes, followed by amplification/PCR cycles as follows: 95°C 10 minuets, then 40 cycles of 95° C for 15 seconds, and 60°C for 1 minute.

The primer-probe sets employed in the expression analysis of each clone, and a summary of the results, are provided below. The complete experimental results are illustrated in FIG. 14. The panel of cell lines employed was identical in all cases except that samples 95 and 96 were gDNA and a melanoma UACC-257 (control), respectively, in the experiments for clone 11696905. The nucleotide sequences of the primer sets used for these clones are as follows:

Clone 11696905.0.47 Primer Set:
Ag 383 (F): 5'-GGCCTCTCCGTACCCTTCTC-3' (SEQ ID NO:25)
Ag 383(R): 5'-AGAGGCTCTTGGCGCAGTT-3' (SEQ ID NO:26)
Ag 383 (P): TET-5'-ACCAGGATCACGACCTCCGCAGG-3'-TAMRA (SEQ ID NO:27)

Primer Set Ag 383 was designed to probe for nucleotides 403-478 in SEPC 3 (clone 11696905.0.47). The results indicate that the clone was prominently expressed in normal cells such as adipose, adrenal gland, various regions of the brain, skeletal muscle, bladder, liver and fetal liver, mammary gland, placenta, prostate and testis. It was also found to be expressed at levels much higher than comparable normal cells in cancers of the kidney and lung, and expressed at levels much lower than comparable normal cells in cancers of the central nervous system (CNS) and breast. These results suggest that SEPC 3 (clone 11696905.0.47), or fragments thereof, may be useful in probing for cancer in kidney and lung, and that the nucleic acid or the protein of clone 11696905.0.47 may be a target for therapeutic agents in such cancers. These nucleic acids and proteins may be useful as therapeutic agents in treating cancers of the CNS and breast.

Clone 16406477.0.206 Primer Set:
Ag 53 (F): 5'-GCCTGGCACGGACTATGTGT-3' (SEQ ID NO:28)
Ag 53 (R): 5'-GCCGTCAGCCTTGGAAAGT-3' (SEQ ID NO:29)
Ag 53 (P): TET-5'-CCATTCCCGCTGCACTGTGACG-3'-TAMRA (SEQ ID NO:30)

SEPC 7 (clone 16406477.0.206) was found to be expressed essentially exclusively in testis cells, with a low level of expression in the hypothalamus, among the cells tested.

Clone 21433858 Primer Set:
Ag 127 (F): 5'-CCTGCCAGGATGACTGTCAATT-3' (SEQ ID
NO:31)
Ag 127 (R): 5'-TGGTCCTAACTGCACCACAGTCT-3' (SEQ ID
NO:32)
Ag 127 (P): TET-5'-CCAGCTGGTCCAAGTTTTCTTCATGCAA-3'-TAMRA (SEQ ID
NO:33)

Probe set Ag 127 targets nucleotides 2524-2601 of SECP1 (clone 21433858). The results show that the clone is expressed principally in normal tissues such as adipose, brain, bladder, fetal and adult kidney, mammary gland, myometrium, uterus, placenta, and testis. In comparison to normal lung tissue, it is highly expressed in a small cell lung cancer, a large cell lung cancer, and a non-small cell lung cancer. Therefore, SECP1 (clone 21433858), or a fragment thereof, may be useful as a diagnostic probe for such lung cancers. The nucleic acids or proteins of SECP1 (clone 21433858) may furthermore serve as targets for the treatment of cancer in these and other tissues.

Clone 21637262.0.64 Primer Set:
Ab5(F): 5'-GTGATCCTCAGGCTGGACCA-3' (SEQ ID NO:34)
Ab5(R): 5'-TTCTGACTGGGCTGCATCC-3' (SEQ ID NO:35)
Ab5(P): FAM-5'-CCAGTGTTTCCTCAGCACAGGGCC-3'-TAMRA (SEQ ID NO:36)

Probe set Ab5 targets nucleotides 1221-1298 in SECP9 (clone 21637262.0.64). The results shown in FIG. 14 demonstrate that SECP9 (clone 21637262.0.64) is expressed in cells from normal tissues including, especially, the salivary gland and trachea, among those cells examined.

### Other Embodiments

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. An isolated polypeptide comprising an amino acid sequence selected from the group consisting of:
(a) a mature form of an amino acid sequence selected from the group consisting of SEQ ID NO: 6;
(b) a variant of a mature form of an amino acid sequence selected from the group consisting of SEQ ID NO: 6; wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NO: 6; and
(d) a variant of an amino acid sequence selected from the group consisting of SEQ ID NO: 6, wherein one or more amino acid residues in said variant differs from the amino acid sequence of said mature form, provided that said variant differs in no more than 15% of amino acid residues from said amino acid sequence.

2. The polypeptide of Claim 1, wherein said polypeptide comprises the amino acid sequence of a naturally-occurring allelic variant of an amino acid sequence selected from the group consisting of SEQ ID NO: 6.

3. The polypeptide of Claim 2, wherein said allelic variant comprises an amino acid sequence that is the translation of a nucleic acid sequence differing by a single nucleotide from a nucleic acid sequence selected from the group consisting of SEQ ID NO: 5.

4. The polypeptide of Claim 1, wherein the amino acid sequence of said variant comprises a conservative amino acid substitution.

5. An isolated nucleic acid molecule comprising a nucleic acid sequence selected from the group consisting of:
(a) a nucleic acid sequence encoding a polypeptide comprising a mature form of an amino acid sequence selected from the group consisting of SEQ ID NO: 6;
(b) a nucleic acid sequence encoding a polypeptide comprising a variant of a mature form of an amino acid sequence selected from the group consisting of SEQ ID NO: 6, wherein one or more amino acid residues in said variant differs from the amino acid sequence of said mature form, provided that said variant differs in no more than 15% of the amino acid residues from the amino acid sequence of said mature form;
(c) a nucleic acid sequence encoding a polypeptide comprising an amino acid sequence selected from the group consisting of SEQ ID NO: 6;
(d) a nucleic acid sequence encoding a polypeptide comprising a variant of an amino acid sequence selected from the group consisting of SEQ ID NO: 6, wherein one or more amino acid residues in said variant differs from the amino acid sequence of said mature form, provided that said variant differs in no more than 15% of amino acid residues from said amino acid sequence;
(e) a nucleic acid fragment encoding at least a portion of a polypeptide comprising an amino acid sequence chosen from the group consisting of SEQ ID NO: 6, or a variant of said polypeptide, wherein one or more amino acid residues in said variant differs from the amino acid sequence of said mature form, provided that said variant differs in no more than 15% of amino acid residues from said amino acid sequence; and
(f) a nucleic acid molecule comprising the complement of (a), (b), (c), (d) or (e).

6. The nucleic acid molecule of Claim 5, wherein the nucleic acid molecule comprises the nucleotide sequence of a naturally-occurring allelic nucleic acid variant.

7. The nucleic acid molecule of Claim 5, wherein the nucleic acid molecule encodes a polypeptide comprising the amino acid sequence of a naturally-occurring polypeptide variant.

8. The nucleic acid molecule of Claim 5, wherein the nucleic acid molecule differs by a single nucleotide from a nucleic acid sequence selected from the group consisting of SEQ ID NO: 5.

9. The nucleic acid molecule of Claim 5, wherein said nucleic acid molecule comprises a nucleotide sequence selected from the group consisting of:
(a) a nucleotide sequence selected from the group consisting SEQ ID NO: 5;
(b) a nucleotide sequence differing by one or more nucleotides from a nucleotide sequence selected from the group consisting of SEQ ID NO: 5, provided that no more than 20% of the nucleotides differ from said nucleotide sequence;
(c) a nucleic acid fragment of (a); and
(d) a nucleic acid fragment of (b).

10. The nucleic acid molecule of Claim 5, wherein said nucleic acid molecule hybridizes under stringent conditions to a nucleotide sequence chosen from the group consisting of SEQ ID NO: 5, or a complement of said nucleotide sequence.

11. The nucleic acid molecule of Claim 5, wherein the nucleic acid molecule comprises a nucleotide sequence selected from the group consisting of:
(a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
(b) an isolated second polynucleotide that is a complement of the first polynucleotide; and
(c) a nucleic acid fragment of (a) or (b).

12. A vector comprising the nucleic acid molecule of Claim 11.

13. The vector of Claim 12, further comprising a promoter operably-linked to said nucleic acid molecule.

14. A cell comprising the vector of Claim 12.

15. An antibody that immunospecifically-binds to the polypeptide of Claim 1.

16. The antibody of Claim 15, wherein said antibody is a monoclonal antibody.

17. The antibody of Claim 15, wherein the antibody is a humanized antibody.

18. A method for determining the presence or amount of the polypeptide of Claim 1 in a sample, the method comprising:
(a) contacting the sample with an antibody that binds immunospecifically to the polypeptide; and
(b) determining the presence or amount of antibody bound to said polypeptide,
thereby determining the presence or amount of polypeptide in said sample.

19. A method for determining the presence or amount of the nucleic acid molecule of Claim 5 in a sample, the method comprising:
(a) contacting the sample with a probe that binds to said nucleic acid molecule; and
(b) determining the presence or amount of the probe bound to said nucleic acid molecule,
thereby determining the presence or amount of the nucleic acid molecule in said sample.

20. A method of identifying an agent that binds to a polypeptide of Claim 1, the method comprising:
(a) contacting said polypeptide with said agent; and
(b) determining whether said agent binds to said polypeptide.

21. A method for identifying an agent that modulates the expression or activity of the polypeptide of Claim 1, the method comprising:
(a) contacting a cell expressing said polypeptide with said agent; and
(b) determining whether the agent modulates expression or activity of said polypeptide,
whereby an alteration in expression or activity of said peptide indicates said agent modulates expression or activity of said polypeptide.

22. A method for modulating the activity of the polypeptide of Claim 1, the method comprising contacting a cell sample expressing the polypeptide of said claim with a compound that binds to said polypeptide in an amount sufficient to modulate the activity of the polypeptide.

23. Use of the polypeptide of Claim 1 for the manufacture of a medicament for treating or preventing a SECP-associated disorder in a subject.

24. Use of the nucleic acid of Claim 5 for the manufacture of a medicament for treating or preventing a SECP-associated disorder in a subject.

25. Use of the antibody of Claim 15 for the manufacture of a medicament for treating or preventing a SECP-associated disorder in a subject.

26. Use according to any of Claims 23-25, wherein the subject is a human.

27. A pharmaceutical composition comprising the polypeptide of Claim 1 and a pharmaceutically-acceptable carrier.

28. A pharmaceutical composition comprising the nucleic acid molecule of Claim 5 and a pharmaceutically-acceptable carrier.

29. A pharmaceutical composition comprising the antibody of Claim 15 and a pharmaceutically-acceptable carrier.

30. A kit comprising in one or more containers, the pharmaceutical composition according to any of Claims 27-29.

31. Use of a therapeutic for the manufacture of a medicament for treating a syndrome associated with a human disease, the disease selected from a SECP-associated disorder, wherein said therapeutic is selected from the group consisting of a SECP polypeptide, a SECP nucleic acid, and a SECP antibody.

32. A method for screening for a modulator of activity or of latency or predisposition to a SECP-associated disorder, said method comprising:
(a) administering a test compound to a test animal at increased risk for a SECP-associated disorder, wherein said test animal recombinantly expresses the polypeptide of Claim 1;
(b) measuring the activity of said polypeptide in said test animal after administering the compound of step (a);
(c) comparing the activity of said protein in said test animal with the activity of said polypeptide in a control animal not administered said polypeptide, wherein a change in the activity of said polypeptide in said test animal relative to said control animal indicates the test compound is a modulator of latency of or predisposition to a SECP-associated disorder.

33. The method of Claim 32, wherein said test animal is a recombinant test animal that expresses a test protein transgene or expresses said transgene under the control of a promoter at an increased level relative to a wild-type test animal, and wherein said promoter is not the native gene promoter of said transgene.

34. A method for determining the presence of or predisposition to a disease associated with altered levels of the polypeptide of Claim 1 in a first mammalian subject, the method comprising:
(a) measuring the level of expression of the polypeptide in a sample from the first mammalian subject; and
(b) comparing the amount of said polypeptide in the sample of step (a) to the amount of the polypeptide present in a control sample from a second mammalian subject known not to have, or not to be predisposed to, said disease,
wherein an alteration in the expression level of the polypeptide in the first subject as compared to the control sample indicates the presence of or predisposition to said disease.

35. A method for determining the presence of or predisposition to a disease associated with altered levels of the nucleic acid molecule of Claim 5 in a first mammalian subject, the method comprising:
(a) measuring the amount of the nucleic acid in a sample from the first mammalian subject; and
(b) comparing the amount of said nucleic acid in the sample of step (a) to the amount of the nucleic acid present in a control sample from a second mammalian subject known not to have, or not to be predisposed to, the disease;
wherein an alteration in the level of the nucleic acid in the first subject as compared to the control sample indicates the presence of or predisposition to the disease.

36. Use of a polypeptide having an amino acid sequence at least 95% identical to a polypeptide comprising an amino acid sequence of SEQ ID NO: 6, or a biologically active fragment thereof, for the manufacture of a medicament for treating a pathological state in a mammal.

37. Use of the antibody of Claim 15 for the manufacture of a medicament for treating a pathological state in a mammal.
